# EUROPÄISCHE PATENTSCHRIFT

(11) **EP 3 060 893 B1**
(45) Veröffentlichungstag und Bekanntmachung des Hinweises auf die Patenterteilung: **18.09.2019**
(21) Anmeldenummer: 14795984.5
(22) Anmeldetag: 27.10.2014
(51) Int. Cl.: A61B 17/00, A61B 5/0215, A61B 5/03, A61B 5/08, A61B 5/20, G01L 9/00, G01L 9/08, G01L 1/24, G01L 11/02, A61B 5/00, A61B 90/00, A61B 90/30, A61B 34/20

(54) **DRUCKMESSVORRICHTUNG**
PRESSURE GAUGE
MANOMÈTRE

(30) Priorität: 25.10.2013 DE 102013111817; 28.02.2014 DE 202014100938 U
(43) Veröffentlichungstag der Anmeldung: 31.08.2016
(73) Patentinhaber: Fibragg Diagnostics GmbH, 60486 Frankfurt am Main (DE)
(72) Erfinder: HECKER, Raoul, 60486 Frankfurt am Main (DE); BARZ, Thomas, 16303 Schwedt (DE); MELLOH, Markus, CH-8400 Winterthur (CH)
(74) Vertreter: Haar, Lucas Heinz Jörn
(86) Internationale Anmeldenummer: PCT/EP2014/073030
(87) Internationale Veröffentlichungsnummer: WO 2015/059311

(56) Entgegenhaltungen:
- EP-A1- 2 491 883
- EP-A2- 1 154 269
- WO-A1-99/58059
- US-A1- 2004 067 000
- US-A1- 2008 021 289
- US-A1- 2008 294 144
- US-A1- 2011 087 112
- VOIGT S ET AL: "Investigations on pressure sensors for medical applications based on fiber Bragg gratings", 2013 TRANSDUCERS & EUROSENSORS XXVII: THE 17TH INTERNATIONAL CONFERENCE ON SOLID-STATE SENSORS, ACTUATORS AND MICROSYSTEMS (TRANSDUCERS & EUROSENSORS XXVII), IEEE, 16 June 2013 (2013-06-16), pages 78-81, XP032499798, DOI: 10.1109/TRANSDUCERS.2013.6626705 [retrieved on 2013-10-09]

## Beschreibung

Die Erfindung betrifft eine Druckmessvorrichtung zur Druckmessung in einem biologischen System mit einem biegeflexibel oder biegeelastisch ausgestalteten Messzellenträger.

Es sind katheterbasierte Systeme bekannt, die bei diagnostischen und therapeutischen Anwendungen verwendet werden. Katheterbasierte optische Systeme umfassen üblicherweise eine Konsole, die beispielsweise tragbar, eine montierbare Ablage oder eine Bodenstation ist. Eine optische Fasersonde, eine Kamera oder ein Katheter ist mit der Konsole verbunden, bisweilen auch kontaktlos, beispielsweise über elektromagnetische Wellen.

Der Katheter wird üblicherweise durch einen Anschlussadapter mit der Konsole verbunden. Häufig ist der Katheter nur für den einmaligen Gebrauch bestimmt. Daher muss der Katheter nach seinem Gebrauch abgenommen werden. Auch wenn der Katheter nicht wegwerfbar ist, muss er zum Reinigen oder Sterilisieren zwischen den einzelnen Gebrauchsphasen abgenommen werden.

Eine ebenfalls katheterbasierte aber nicht bildgebende sondern manometrische Messung wird dazu verwendet, Funktionsstörungen zum Beispiel in Organen wie der Speiseröhre und dem Darm festzustellen. Hierbei wird eine Druckmessung in dem Organ durchgeführt und mit einem Standard-Referenzdruck verglichen, wobei auf mögliche Funktionsstörungen geschlossen werden kann.

Bei der Perfusionsmanometrie wird ein ein- oder mehrlumiger Katheter in die Speiseröhre eingebracht, wobei die Kanäle des Katheters mit Flüssigkeit bei einer möglichst konstanten Durchflussrate durchströmt sind. Druckschwankungen in der Speiseröhre können durch Veränderungen im Arbeitsdruck ermittelt werden.

Ferner ist die Verwendung eines mikroelektronischen Sensors bekannt, der beispielsweise in einen Katheter integriert sein kann und zur Messung der Temperatur oder des Druckes genutzt wird. Temperatursensoren verwenden im Allgemeinen auf Halbleitersubstraten basierende integrierte dotierte Widerstände oder Dioden. Die elektrischen Eigenschaften dieser Bauelemente haben eine wohldefinierte Temperaturabhängigkeit, was für die Funktion als Sensor ausgenutzt wird. Die mikroelektronischen Drucksensoren werden bisher allerdings ausschließlich für die hydrostatische Druckmessung verwandt, zum Beispiel als Sonde zur Messung des intrakraniellen Druckes (des Schädelinnendruckes).

Weiterhin ist eine pneumatische Druckmessung mit einer intraventrikulären, subduralen, intraparenchymalen oder epiduralen Sonde möglich. Das hierbei verwendete gasgefüllte Kammersystem ist ein Hohlkörper aus Kunststoff, der über einen Schlauch mit einem Druckaufnehmer verbunden ist. Der Druckaufnehmer befindet sich zusammen mit der Messelektronik und einer Vorrichtung zur Füllung des Kammersystems in einem ICP (intracranial pressure)-Monitor. Zur Hirndruckmessung wird das gasgefüllte Kammersystem im Ventrikel oder im Parenchym platziert. Zur epiduralen Druckmessung / Hirndruckmessung wird das Kammersystem auf der Dura mater des Patienten platziert. Der intrakranielle Druck wird über die dünne Wand des Kammersystems auf die Luft in dem Kammersystem übertragen und vom Druckaufnehmer in elektrische Signale umgesetzt.

Im Stand der Technik sind außerdem Tip-Sensoren bekannt, die aus einem Katheter mit einem Sensorelement an dessen Spitze (englisch: "tip") bestehen. Die eigentliche Druckmessplatte des Sensorelementes umfasst zu einer vollständigen oder teilweisen geschlossenen Wheatstonesche Messbrücke. Die Brückenschaltung wird mittels einer im Katheter liegenden Drahtleitung mit Konstantstrom gespeist. Wird die Druckmessplatte mit Druck belastet, so kommt es auf Grund der mechanischen Spannung in der Platte und dem piezoresistiven Effekt zu einer Änderung der Brückenausgangsspannung. Der Sensor findet beispielsweise in neurochirurgischen Anwendungen Verwendung. Sie erfahren Einschränkungen durch die recht klobige Bauweise, die durch die Abmessungen der erhältlichen Piezo-Sensoren bestimmt ist.

Ferner sind Vorrichtungen bekannt, mit denen beispielsweise ein Druck in der Speiseröhre gemessen werden kann. Hierbei ist ein Bragg-Gitter in einen Lichtleiter integriert. Es sind verschiedene Methoden bekannt, solche Bragg-Gitter (auch bekannt als Faser-Bragg-Gitter) herzustellen. Beispielsweise werden Femtosekundenlaser verwendet, mit deren Hilfe die Gitterstrukturen Punkt für Punkt in den Faserkern eingebracht werden. Eine Entfernung des Fasermantels (Coating) entfällt bei diesem Verfahren. Im Unterschied dazu muss bei einer UV-Belichtung mit Phasenmaske oder einem Excimer Laser Punkt für Punkt der Fasermantel entfernt werden. Durch die wiederholte Aufbringung des Fasermantels nach der Belichtung entsteht eine mechanisch leicht brüchige Stelle. Vorzugsweise wird daher die UV-Belichtung direkt am Faserziehturm durchgeführt, bevor das Coating aufgebracht wird. Bei Verwendung von UV-Lichtquellen ist ferner eine Dotierung des Faserkems mit Germanium notwendig. Die bekannten Vorrichtungen zur Druckmessung in der Speiseröhre weisen mitunter ein Bragg-Gitter auf, das an einen beweglichen Wandabschnitt gekoppelt ist. Zudem sind starre Befestigungselemente an dem Lichtleiter seitlich zu dem Bragg-Gitter angebracht. Durch einen auf den beweglichen Wandabschnitt einwirkenden Druck bewegt sich der bewegliche Wandabschnitt in Richtung des Bragg-Gitters und übt wiederum eine Kraft auf dieses aus, so dass der Lichtleiter in einen von den beweglichen Wandabschnitten und den starren Befestigungselementen begrenzten Freiraum bewegt wird. Diese Bewegung oder Dehnung des Lichtleiters resultiert in einer optischen Antwort des Bragg-Gitters. Nachteilig bei der bekannten Vorrichtung ist, dass die Fertigung sehr aufwendig und kostenintensiv ist. Aufgrund der um das Bragg-Gitter angeordneten Bestandteile hat die Vorrichtung einen recht großen Durchmesser, was ihre Verwendung stark einschränkt. Außerdem kann die Vorrichtung nicht hitzesterilisiert werden, da die Lichtleiter mit stoffschlüssigen Verbindungsmitteln an den beweglichen Wandabschnitten, beziehungsweise den starren Befestigungsmitteln festgelegt sind. Durch Temperatureinwirkung oder andere physikalische oder chemische Maßnahmen kann sich der Lichtleiter von den Verbindungsmitteln lösen, was wiederum zu einem irreparablen Schaden und schließlich Funktionsverlust der Vorrichtung führt.

US 2004/0067000 A1 offenbart ein bildgebendes System mit mindestens einer optischen Faser. An dem oder nahe dem distalen Ende der Faser liegt ein Faser-Bragg-Gitter vor, das Licht zu einem fotoakustischem Wandler sendet. Der Wandler generiert hieraus Ultraschall. Reflektierter Ultraschall wird dann von einem weiteren in der optischen Faser vorliegenden Faser-Bragg-Gitter detektiert und durch die Faser an eine Empfangsvorrichtung weitergeleitet, die dann wiederum ein 2-D- oder 3-D-Bild erzeugt.

Aus WO 99/58059 A1 ist eine optische-akustische Vorrichtung bekannt, die eine optische Faser mit mindestens einem Faser-Bragg-Gitter und einem piezoelektrischen Element aufweist.

US 2008/021289 A1 offenbart ein medizinisches Gerät für die Implantation in Körpergewebe. Das implantierbare medizinische Gerät besteht aus einem Gehäuse und einem mit dem Gehäuse gekoppelten Kopfteil. Im Kopfteil befindet sich eine Kavität, in der ein akustischer Wandler zur Übertragung akustischer Wellen aufgenommen ist. Der Wandler kann zum Beispiel aus einem piezoelektrischem Material bestehen.

Aus EP 1 154 269 A2 ist Ultraschallempfänger bekannt, umfassend eine Ultraschalldetektoreinheit mit einer Mehrzahl von Ultraschalldetektorelementen, die in einer zweidimensionalen Anordnung platziert sind, um Licht auf der Grundlage einer aufgebrachten Ultraschallwelle zu modulieren und einen schmalbandigen Bandpassfilter. Ferner umfasst der Empfänger einen Fotodetektor zum Detektieren des Ausgangslichts von den mehreren Ultraschalldetektorelementen, um Detektorsignale auszugeben. Jedes der mehreren Ultraschalldetektorelemente weist eine Bragg-Gitterstruktur auf, wobei der schmalbandige Bandpassfilter eine Bragg-Gitterstruktur aus den gleichen Werkstoffen wie die Bragg-Gitterstruktur der mehreren Ultraschalldetektorelemente aufweist. Die Bragg-Gitterstruktur des schmalbandigen Bandpassfilters und die Bragg-Gitterstruktur der mehreren Ultraschalldetektorelemente sind thermisch gekoppelt.

US 2008/294144 A1 offenbart einen Berührungssensor-Katheter mit einer Dehnungssensoranordnung, die die Größe und Richtung einer Kraft auflösen kann, die auf eine distale Extremität des Katheters ausgeübt wird, wobei die Dehnungssensoranordnung im Wesentlichen unempfindlich gegenüber Temperaturänderungen in der Masse ist. Der Sensor umfasst eine verformbare Struktur mit einer Vielzahl von damit verbundenen optischen Fasern, wie z.B. Fabry-Perot-Resonatoren, die sich in der operativen Länge verändern, wenn eine Kraft auf die verformbare Struktur ausgeübt wird.

EP 2 491 883 A1 beschreibt einen Katheter mit in einen distalen Abschnitt integrierten Kraftsensoren, die zur Messung des Betrages und der Richtung einer auf den distalen Abschnitt wirkenden äußeren Kraft ausgebildet sind. In dem Katheter ist eine einzelne FBG-Faser vorgesehen, welche drei Kraftsensorbereiche umfasst, die die zur gemeinsamen Messsignalverarbeitung zusammenwirkenden Kraftsensoren bildet.

US 2011/087112 A1 offenbart eine Vorrichtung mit in einem distalen Ende angeordnete, faseroptische Kontaktkraftsensoren. Die Sensoren sind so konfiguriert, dass sie mit einer programmierten Verarbeitungslogik gekoppelt sind, die einen Kraftvektor berechnet, der auf erkannte Änderungen der optischen Eigenschaften der faseroptischen Kontaktkraftsensoren reagiert, die sich aus der Ablenkung der distalen Extremität ergeben, die sich wiederum aus dem Kontakt der Vorrichtung mit einer Struktur ergibt. Die Sensoren können zum Beispiel als Faser-Bragg-Sensoren oder Fabry-Perot-Interferometer ausgestaltet sein.

Die wissenschaftliche Publikation VOIGT S ET AL: "Investigations on pressure sensors for medical applications based on fiber Bragg gratings" (2013 TRANSDUCERS & EUROSENSORS XXVII: THE 17TH INTERNATIONAL CONFERENCE ON SOLID-STATE SENSORS, ACTUATORS AND MICROSYSTEMS (TRANSDUCERS & EUROSENSORS XXVII), IEEE, 16. Juni 2013 (2013-06-16), Seiten 78-81, XP032499798) offenbart einen Drucksensor-Katheter mit einem Polymerschutzmantel basierend auf einen Faser-Bragg-Gitter (FBG), wobei bis zu 32 FBG Sensoren in einer Glasfaser integriert und die Messergebnisse ausgelesen werden können.

Nachteilig bei den bekannten Druckmessgeräten ist vorallem, dass die Druckmessung nur an einem Punkt erfolgt und somit keine räumlich-kontinuierliche Messung möglich ist. Außerdem sind die bekannten Druckmessgeräte oftmals nicht sterilisierbar, sondern als Einwegartikel vorgesehen, da sie nur schwer oder gar nicht zu reinigen sind, was wiederum mit hohen Kosten verbunden ist. Ein weiterer Nachteil ist, dass die Druckmessgeräte nicht universell einsetzbar sind. Auch erliegen im klinischen Einsatz Konstruktionen mit metallischen Komponenten starken Einschränkungen durch ihre Eigenschaft, bei magnetresonanztomographischen (MRT) Untersuchungen elektrische Induktion zu zeigen und damit die MRT-Untersuchung zu stören und zudem elektrischen Strom, Dislokation und Hitze im untersuchten biologischen System zu entwickeln. Auch auf Röntgenstrahlen basierende bilddiagnostische Verfahren, zum Beispiel CT-Untersuchungen, können durch metallische Komponenten gestört werden. Druckmessgeräte, die bisher ggf. ohne metallische Komponenten auskommen (zum Beispiel pneumatische), haben wiederum in den Nachteil, dass ihre Form durch Biegungen im eingebrachten Organismus für den Untersucher im klinischen Einsatz oft nur gemutmaßt werden kann.

Der Erfindung liegt die Aufgabe zugrunde, ein Druckmessgerät bereitzustellen, welches nicht die Nachteile und Mängel der bekannten Druckmessgeräte aufweist.

Erfindungsgemäß wird diese Aufgabe durch eine Druckmessvorrichtung zur Druckmessung in einem biologischen System mit einem biegeflexibel oder biegeelastisch ausgestalteten und aus mindestens einem Lichtwellenleiter bestehenden Messzellenträger gelöst, der ein Auswerteeinheit-nahes Ende und ein Auswerteeinheit-fernes Ende aufweist, wobei in dem Messzellenträger mindestens zwei zueinander beabstandet angeordnete Faser-Bragg-Gitter Sensoren vorliegen und in dem Auswerteeinheit-fernen Ende ein Fabry-Perot-Interferometer angeordnet ist, welches sich aus mindestens einem Ende des Auswerteeinheit-fernen Endes des Messzellenträgers sowie mindestens einer deformierbaren verspiegelten Membran zusammensetzt, wobei das Fabry-Perot-Interferometer im gleichen Wellenlängenfenster wie die Faser-Bragg-Gitter Sensoren auswertbar ist. Die Faser-Bragg-Gitter Sensoren können im Sinne der Erfindung auch als Messzellen bezeichnet werden. Die Druckmessvorrichtung ist biegeflexibel oder biegeelastisch, insbesondere radial dehnbar oder stauchbar ausgestaltet, so dass eine radial einwirkende Kraft zu einer Verformung des Messzellenträgers führt. Dem Fachmann ist bekannt, dass eine Ursprungsform des Messzellenträgers bei Wegfall der einwirkenden Kraft wieder erreicht werden kann und somit ein biegeelastischer oder plastischer Messzellenträger vorliegt. Es kann jedoch auch vorteilhaft sein, wenn die Ursprungsform nach Wegfall der Krafteinwirkung nicht wieder eingenommen wird und somit ein biegeflexibler Messzellenträger besteht. Die biegeelastische oder biegeflexible Ausgestaltung des Messzelienträgers kann durch die Materialwahl des Messzellenträgers beeinflusst werden, wobei der Fachmann weiß, dass auch ein hartes Material durch eine entsprechende Krafteinwirkung elastisch verformt werden kann. Durch die biegeelastische oder biegeflexible Ausgestaltung des Messzellenträgers wird erreicht, dass äußere Krafteinwirkungen eine radiale Dehnung oder Stauchung und somit eine Verformung des Messzellenträgers bewirken. Mit der erfindungsgemäßen Vorrichtung ist vorteilhafterweise eine Erfassung von Druckmessergebnissen über einen zeitlichen Verlauf möglich, wobei zusätzlich eine Lageveränderung des Messzellenträgers oder des biologischen Systems (insbesondere eines Patienten) erfolgen kann (beispielsweise sitzend / liegend / stehend, Bauch- / Rückenlage, oder bestimmte Bewegungen und Belastungen).

Die Messzellen sind in dem Messzellenträger beabstandet zueinander angeordnet. Auch wenn die Krafteinwirkung auf den Messzellenträger gering ist, bewirkt sie eine Verformung des Messzellenträgers und somit eine Dehnung der Messzellen. Die einwirkende Kraft kann somit durch die Messzellen als Druck erfasst werden.

Dadurch, dass die Messzellen beabstandet zueinander angeordnet sind, können mehrere, gegebenenfalls unterschiedliche Drücke gemessen werden. Hierdurch ist auch eine räumlich-/zeitlich-kontinuierliche Druckmessung in einem biologischen System möglich. Es kann jedoch auch vorteilhaft sein, wenn eine Messzelle Drücke an zwei oder mehreren verschiedenen Messstellen in dem biologischen System erfasst. Die Druckmessvorrichtung kann beispielsweise durch eine Lageverschiebung von der ersten zu der zweiten Messstelle geführt werden und an der ersten Messstelle einen ersten und an der zweiten Messstelle einen zweiten Druck messen. Dies bringt überraschende Vorteile mit sich, da einer der gemessenen Drücke als Referenzwert herangezogen werden kann. Somit können Messartefakte bei der Druckmessung berücksichtigt und mittels Referenzwert eliminiert werden.

Es kann auch vorteilhaft sein, wenn die Druckmessvorrichtung Drücke an einer Messstelle in zeitlicher Abfolge bestimmt, das heißt, eine Messzelle kann insbesondere mindestens zwei Drücke an einer Messstelle in zeitlicher Abfolge erfassen, wobei die zweite Messzelle als Referenzmesszelle verwendet wird.

Ferner können die Messzellen Drücke nacheinander oder gleichzeitig messen. Dies kann insbesondere dann vorteilhaft sein, wenn mehr als zwei, bevorzugt mehr als fünf, besonders bevorzugt mehr als zehn Messzellen in dem Messzellenträger beabstandet zueinander vorliegen. Hierdurch können über eine große Länge des Messzellenträgers Drücke bestimmt werden. Es ist möglich, dass die Messzellen die Drücke gleichzeitig oder nacheinander erfassen.

Vorteilhafterweise kann die Druckmessvorrichtung einfach und sicher mit einer axial einwirkenden Schubkraft oder Druckbelastung in ein biologisches System eingebracht (eingeschoben) werden, wobei die Druckmessvorrichtung im Wesentlichen keine axiale Stauchung erfährt. Es hat sich herausgestellt, dass die Druckmessvorrichtung vorteilhafterweise keine nennenswerte Stauchung erfährt und die nicht-nennenswerte Stauchung der Druckmessvorrichtung nach Entlastung, beispielsweise Wegfall der wirkenden Schubkraft, ausgeglichen wird. Die Druckmessvorrichtung kann somit im Sinne der Erfindung mit Bezug auf eine axial einwirkende Schubkraft oder Druckbelastung insbesondere als im Wesentlichen schubstabil bezeichnet werden. Die Länge der Druckmessvorrichtung wird durch die axial einwirkenden Kräfte im Wesentlichen nicht verändert. Das bringt den Vorteil mit sich, dass die Druckmessvorrichtung einfach in ein biologisches System einführbar und in diesem gut führbar beziehungsweise manövrierbar ist und zudem die Verletzungsgefahr des biologischen Systems reduziert wird.

Auch durch eine axial einwirkende Zugbelastung wird im Wesentlichen keine Verformung der Druckmessvorrichtung bewirkt, so dass auch eine einfache und sichere Entfernung der Druckmessvorrichtung aus dem biologischen System möglich ist.

Ein biologisches System bezeichnet im Sinne der Erfindung insbesondere ein Lebewesen, insbesondere einen Menschen, ein Nutztier oder ein Haustier. Es zählen zu den Nutztieren bevorzugt alle in der Landwirtschaft genutzten Haustierrassen, gleich ob diese Nutztiere für Ernährungszwecke oder als Last- oder Zugtiere dem Menschen einen Nutzen erbringen. Weiterhin zählen zu den Nutztieren insbesondere alle Tierarten und Rassen, die vorrangig wegen ihrem Fell als Wolllieferanten domestiziert wurden und ebenso Tierarten und Haustierrassen, die dem Menschen auf anderer Art und Weise als nützlich erscheinen. Als Haustiere werden insbesondere alle domestizierten Tierarten und Tierrassen bezeichnet. Die erfindungsgemäße Druckmessvorrichtung kann universell in biologischen Systemen zur Druckmessung verwendet werden.

Die Druckmessvorrichtung ist insbesondere als fadenförmiges oder schlauchförmiges Gebilde ausgeformt, wobei vorteilhafterweise der Messzellenträger ein erstes und zweites Ende aufweist. Das erste Ende der Druckmessvorrichtung kann als Auswerteeinheit-nahes Ende bezeichnet werden. Das zweite Ende des Messzellenträgers, das heißt das Ende, welches in ein biologisches System eingeführt werden kann, wird im Sinne der Erfindung als Auswerteeinheit-fernes Ende bezeichnet. Das Auswerteeinheit-ferne Ende kann in einer Ausgestaltung der Erfindung eine atraumatische Spitze oder Führungsstruktur aufweisen. Bei der Führungsstruktur kann es sich beispielweise um einen festinstallierten oder herausziehbaren und / oder hineinschiebbaren Führungsdraht oder um eine Struktur handeln, die geeignet ist, das Einführen in abgehende oder verzweigende biologische Strukturen zu erleichtern.

Die atraumatische Spitze ist bevorzugt aus einem solchen Kunststoffmaterial gefertigt, dass es zu keiner Verletzung des biologischen Systems beim Einführen der Druckmessvorrichtung kommt. Bevorzugte Kunststoffe sind Polyoxymethylen, Polyurethan und / oder Polyamid. Es hat sich in Experimenten gezeigt, dass die Spitze bei dem Einführen in das biologische System keine Verletzung des biologischen Systems verursacht. Um das Einführen zusätzlich zu verbessern, kann die Spitze in einer bevorzugten Ausgestaltung eine Verdickung oder eine Abrundung aufweisen. Auch kann für bevorzugte Anwendungen, beispielsweise einer Druckmessung mittels der erfindungsgemäßen Druckmessvorrichtung in Herzkranzgefäßen, eine spezielle Verlängerung der Druckmessvorrichtung aus einem flexiblen Material vorhanden sein, das beispielsweise initial durch eine innenliegende Schienung (Mandrin) gerade ist, und dann beim Herausziehen der Schienung eine gebogene Form einnimmt.

Die Einbringung der Druckmessvorrichtung erfolgt vorzugsweise über Einstiche in das biologische System unter Verwendung üblicher Hohlnadeln (Trokar). Bei Einstichen in arterielle Systeme mit höherem Druck können diese Hohlnadeln mit Schleusensystemen kombiniert ausgeführt sein. Diese Nadeln verbleiben entweder an der Einstichstelle oder werden zurückgezogen und durch einen innenliegenden Schlauch ersetzt. Ebenso kann die Druckmessvorrichtung vorteilhafterweise aber auch über natürlich vorkommende Öffnungen im biologischen System oder durch Operationen eingebracht werden.

Die Druckmessvorrichtung wird anschließend bevorzugt wieder herausgezogen. Ebenso kann sie aber auch vorübergehend oder auch ständig im biologischen System verbleiben. Ebenso kann die Druckmessvorrichtung auch durch eine Operation eingebracht und gegebenenfalls anschließend, unter Umständen auch durch eine weitere Operation, wieder ausgebracht werden.

An dem Auswerteeinheit-nahen Ende können eine Speisungs- und Auswerteelektronik, eine Datenverarbeitungseinheit und / oder eine Steuer- und Regelungseinheit, sowie die ggf. notwenigen Energie- und Kommunikationsversorgungen angeschlossen sein. Hierdurch sind effiziente und fehlerminimierte Erfassungen und Berechnungen des Druckes beziehungsweise der Drücke und / oder von Druckunterschieden und / oder Katheter-Biegungen und / oder Temperaturen möglich.

Der Messzellenträger ist ein Lichtwellenleiter (LWL), insbesondere eine Einmodenfaser (englisch: "monomode fiber"). Der
Messzellenträger kann auch ein elektrisch leitfähiges Kabel umfassen. Der LWL ist vorzugweise eine polymere optische Faser oder eine Glasfaser. LWL übertragen Licht beziehungsweise Lichtsignale über weite Strecken. Der erfindungsgemäße LWL kann aus Quarz, Glas oder einem Kunststoff, insbesondere einer polymeren optischen Faser gefertigt sein.

Der LWL kann einen zentralen Bereich aufweisen, der auch als Kern bezeichnet wird, wobei ein LWL auch mehrere Kerne aufweisen kann. Der Kern besteht vorzugsweise aus einem Material mit einem anderen, beispielsweise höheren, Brechungsindex als der den zentralen Bereich oder Kern umgebenden ebenfalls aus einem optisch leitfähigen Material gefertigten Mantel. An den Grenzflächen von Kern und Mantel des LWL finden optische Reflexionen statt, so dass der Lichtstrahl weitgehend verlustfrei um jede Biegung geleitet wird. Der Mantel ist das ebenfalls optisch transparente Material, an dem die Reflexion vorzugsweise stattfindet. Der Mantel kann aus einem Material bestehen, welches beispielsweise einen niedrigeren Brechungsindex besitzt als der Kern. Mantel und Kern bestehen bevorzugt aus dielektrischen Materialien, die nichtmetallisch und nichtleitend sind. Es ist auch eine Druckmessvorrichtung vorgesehen, die mehrere Schichten, das heißt Mantelschichten, aufweist. Hierbei können die verschiedenen Schichten aus unterschiedlichen Materialien bestehen und somit auch unterschiedliche Materialeigenschaften besitzen. Dies kann beispielsweise bei bestimmten Anwendungen, bei denen eine gewisse Flexibilität der Druckmessvorrichtung verlangt wird, von Vorteil sein. Weiterhin kann die Flexibilität der Druckmessvorrichtung auch dadurch geändert werden, dass ein Versteifungsmittel in diese eingebracht wird. Das Versteifungsmittel kann beispielsweise als Führungsmittel ausgestaltet sein und in ein Lumen, insbesondere ein Arbeitskanal, der Druckmessvorrichtung eingeführt werden. Das Versteifungsmittel kann diesbezüglich unterschiedliche Steifigkeiten aufweisen, so dass in Abhängigkeit von der gewünschten Flexibilität der Druckmessvorrichtung das Versteifungsmittel gewählt werden kann. Zudem kann die Empfindlichkeit der Druckmessvorrichtung durch die Kombination verschiedener Mantelschichteigenschaften oder verschiedener Mantelschichten mit jeweils verschiedenen Eigenschaften gesteigert, verringert, gerichtet und / oder sonstig angepasst werden.

In einer Ausgestaltung der Erfindung ist vorgesehen, dass der Messzellenträger eine äußere, aus einem biokompatiblen Material bestehende, Schutzschicht aufweist.

Hierbei kann es sich beispielsweise um eine Kunststoffbeschichtung handeln, die als mechanischer Schutz auf der Oberfläche des Mantelmaterials aufgebracht ist und biokompatibel ausgestaltet ist. Die Schutzschicht kann zum Beispiel um die Mantelschicht extrudiert sein. Für die vorteilhafte Ausführungsform können Kunststoffe aus der Gruppe abgewandelte Naturstoffe, synthetische Kunststoffe (Polykondensate, Polymerisate, Polyaddukte), Duroplaste, und / oder ungesättigte Polyesterharze, umfassend Cellulosenitrat, Celluloseacetat, Cellulosemischester, Celluloseether, Polyamid, Polycarbonat, Polyester, Polyphenylenoxid, Polysulfon, Polyvinylacetal, Polyethylen, Polypropylen, Poly-1-buten, Poly-4-methyl-1-penten, lonomere, Polyvinylchlorid, Polyvinylidenchlorid, Polymethyl-methacrylat, Polyacrylnitril, Polystyrol, Polyacetal, Fluor-Kunststoffe, Polyvinylalkohol, Polyvinylacetat, Poly-p-xylylen, lineare Polyurethane, chlorierte Polyether, Casein-Kunststoffe, vernetzte Polyurethane, Silicon, Polyimid, und/ oder Polybenzimidazol verwendet werden. Besonders bevorzugt sind Duromere, Elastomere oder Thermoplaste. Versuche haben gezeigt, dass eine Schutzschicht oder Schutzhülle aus einem thermoplastischen Kunststoff besonders vorteilhaft ist, da die Messzellenträger zum einen gut geschützt sind und zum anderen einwirkende Kräfte, insbesondere zu messende Drücke im Wesentlichen ohne Verluste an die Messzellenträger, beziehungsweise die Messzellen weitergeleitet werden. Die Vorteile der bevorzugten Kunststoffe sind deren Biokompatibilität und hohe Flexibilität im extrudierten Zustand. Zudem sind die hieraus hergestellten Druckmessvorrichtungen sterilisierbar. Thermoplaste sind vorzugsweise ausgewählt aus der Gruppe umfassend Acrylnitril-Butadien-Styrol (ABS), Polyamide (PA), Polylactat (PLA), Polymethylmethacrylat (PMMA), Polycarbonat (PC), Polyethylenterephthalat (PET), Polyolefine, insbesondere Polyethylen (PE) und Polypropylen (PP), Polystyrol (PS), Polyetheretherketon (PEEK), Polyacetale, Polyvinylchlorid (PVC), Celluloseacetobutyrat (CAB), Celluloseacetopropionat (CAP) und Styrol-Acrylnitril (SAN). Überraschenderweise können auch Poly(organo)siloxane verwendet werden.

Bei einem Bruch des LWLs kann die Schutzschicht verhindern, dass Einzelteile von der Druckmessvorrichtung abbrechen und beispielsweise in dem biologischen System zu Verletzungen führen oder unbeabsichtigt verbleiben. Als biokompatibel wird im Sinne der Erfindung insbesondere ein Werkstoff des Messzellenträgers bezeichnet, der im direkten Kontakt mit dem biologischen System keinen nachteiligen Einfluss auf dessen Stoffwechsel oder biologische Funktionalität ausübt.

Die erfindungsgemäße Druckmessvorrichtung weist Faser-Bragg-Gitter-Sensoren (FBG-Sensoren) auf. In einer Ausgestaltung der Erfindung kann vorgesehen sein, dass der Messzellenträger einen piezoelektrischer Sensor in der ersten und / oder zweiten Schicht aufweist, so dass neben den FBG-Sensoren piezoelektrische Sensoren zur Druckmessung in der Druckmessvorrichtung vorliegen. In einer weiteren Ausgestaltung der Druckmessvorrichtung liegen in dem Messzellenträger FBG-Sensoren und piezoelektrische Sensoren vor, wobei die Sensoren in der gleichen Schicht oder in unterschiedlichen Schichten des Messzellenträgers vorliegen. FBG-Sensoren oder piezoelektrische Sensoren werden im Sinne der Erfindung als Messzellen bezeichnet. FBG-Sensoren sind optische Sensoren, die eine spezifische, durch die Gitterstruktur jeweils bedingte, Licht-Wellenlänge reflektieren, die durch Temperatur- und / oder mechanische Kräfte verändert wird. Eine mechanische Dehnung oder Stauchung des Messzellenträgers, insbesondere des LWLs führt zu einer Dehnung oder Stauchung der Gitter und durch den photoelastischen Effekt zu einer Veränderung der von dem jeweiligen FBG-Sensor reflektierten Wellenlänge. FBG-Sensoren erfassen insbesondere radiale Dehnungen des Messzellenträgers. Es hat sich in Versuchen gezeigt, dass der erfindungsgemäße Messzellenträger im Wesentlichen eine konstante Länge aufweist und durch eine axial wirkende Krafteinwirkung im Wesentlichen nicht gestaucht wird. Somit erfassen die erfindungsgemäßen Messzellen, insbesondere die FBG-Sensoren, im Ergebnis überwiegend Dehnungen durch radial auf den Messzellenträger einwirkende Kräfte.

FBG-Sensoren können nach den im Stand der Technik bekannten Methoden hergestellt werden. Wird Licht mit einem breiten Spektrum in einen LWL mit FBG-Sensoren eingekoppelt, erfolgt eine spektral schmalbandige Reflexion an den FBG-Strukturen. Die Reflexionswellenlänge beziehungsweise Bragg-Wellenlänge hängt hierbei von der Gitterperiode der jeweiligen FBG-Sensoren ab. FBG-Sensoren können bevorzugt im Kern des LWLs, als auch im Mantel, das heißt in unterschiedlichen Schichten der Druckmessvorrichtung eingebracht, vorliegen.

Somit reflektiert ein FBG-Sensor vorteilhafterweise eine spezielle Lichtfrequenz, während alle übrigen Frequenzen nahezu unverändert übertragen werden. Da die Bragg-Wellenlänge eine Funktion des Abstands zwischen den Gittern ist, können FBG-Sensoren mit verschiedenen Bragg-Wellenlängen in einem Messzellenträger, insbesondere einem LWL verwendet werden, so dass verschiedene Wellenlängen des Lichts reflektiert werden. Eine Änderung der Temperatur und / oder Dehnung wirken sich sowohl auf den effektiven Brechungsindex als auch auf die Gitterperiode eines FBG-Sensors aus und führen vorteilhafterweise zu einer Veränderung der reflektierten Wellenlänge. Da ein FBG-Sensor sowohl auf Dehnung als auch auf Temperatur reagiert, können vorteilhafterweise beide Einflussfaktoren berücksichtigt werden. Hierbei kann vorgesehen sein, dass die Messzellen der Druckmessvorrichtung entweder einzeln druckmessgerätspezifisch oder serienbauartspezifisch auf ihr Temperatur- und Druckverhalten kalibriert werden.

Durch die geringen, beziehungsweise vernachlässigbaren Leitungsverluste und auch durch die kostengünstige Beschaffenheit kann ein großer, zum Beispiel 1 m oder auch 5 m oder gar mehr als 10 m großer Abstand durch eine lange Leitung zwischen den Messzellen und dem Auswerteeinheit-nahen Ende des Druckmessgerätes erreicht werden, was insbesondere Vorteile für die Handhabung mit sich bringt.

Es kann zur Temperaturmessung und / oder zur quantitativen Erfassung der Temperatureinflüsse auf die gemessene Verschiebung der Bragg-Wellenlänge bevorzugt sein, dass mindestens ein Faser-Bragg-Gitter Sensor in einem axial nicht flexiblen Bereich des Messzellenträgers als Referenzmesszelle vorliegt. Diese Messzelle, insbesondere ein FBG-Sensor, kann als Referenzmesszelle für den Einfluss der Temperatur auf den FBG-Sensor, beziehungsweise auf die Druckmessvorrichtung herangezogen werden. Um einen Bereich des Messzellenträgers unflexibel zu gestalten, kann es vorteilhaft sein, den Messzellenträger, insbesondere den LWL, in dem Bereich der Referenzmesszelle mit einer nicht radial flexiblen Schicht, zum Beispiel mit einer Metallschicht oder auch einem harten Kunststoff zu ummanteln. Durch die Ummantelung kann erreicht werden, dass der FBG-Sensor keinen Biege-, Spannungs-, Druck- oder Torsionskräften ausgesetzt ist und so lediglich die Temperatur Einfluss auf die Lichtreflexion des FBG-Sensors ausübt. Der Einfluss der Temperatur auf die Reflexion des FBG-Sensors kann mit dem Einfluss eines Druckes auf einen zu der Referenzmesszelle beabstandeten FBG-Sensor abgeglichen werden, so dass als Ergebnis lediglich der Einfluss des Druckes auf die Reflexion erfasst wird. Ebenso kann der Temperatureinfluss in gewissen Grenzen durch rechnerische Verfahren eliminiert werden.

Vorteilhafterweise können mehrere in dem Messzellenträger vorliegende Faser-Bragg-Gitter-Sensoren unterschiedliche Resonanzeigenschaften aufweisen, das heißt, es können in einen LWL seriell mehrere FBG-Sensoren mit unterschiedlicher Bragg-Wellenlänge eingebracht werden. Die jeweiligen FBG-Sensoren können dann individuell mit Hilfe eines Wavelength Division Multiplexing (WDM) - Verfahrens adressiert beziehungsweise analysiert werden. Diese spektrale Analyse kann beispielsweise über ein Spektrometer, spektrale Filter oder ein durchstimmbares Fabry-Perot-Etalon erfolgen. Ein alternatives System zum Auslesen der FBG-Sensoren ist die Verwendung eines schmalbandigen durchstimmbaren Lasers als Lichtquelle und einer Photodiode als Detektor. Auch bei Lichtintensitätsverlusten beziehungsweise -dämpfungen im LWL liefern FBG-Sensoren genaue Ergebnisse. Auch Biegungen des LWLs durch die Struktur des biologischen Systems oder durch die Art der Anwendung stören nur unwesentlich die Übermittlung von Informationen aus den FBG-Sensoren. Die Anzahl und Position der FBG-Sensoren kann vorzugsweise für das Messobjekt beziehungsweise dem biologischen System so konzipiert werden, dass eine optimale Erfassung der räumlichen Druckverteilung am Messobjekt erfolgen kann und so auch mögliche Störungsquellen kompensiert werden können.

Die Größe der jeweiligen FBG-Sensoren innerhalb des LWLs, also ihre Länge innerhalb des LWLs, kann in einer bevorzugten Ausführungsform jeweils gleich groß sein, oder die Längen können, angepasst auf die Anwendung, auch variieren. So kann beispielsweise der Untersucher-fernste FBG-Sensor an dem zweiten Ende der Druckmessvorrichtung auch eine besondere Ausgestaltung aufweisen. Die Reflektivität der individuellen FBG - Sensoren kann produktionstechnisch insbesondere so konzipiert und gefertigt werden, dass die Amplitude der reflektierten Signale der FBG-Sensoren am Ausgang der Druckmessvorrichtung etwa gleich groß ist. In einer weiteren vorteilhaften Ausführungsform kann die Länge der jeweiligen FBG-Sensoren den Dämpfungseigenschaften des jeweiligen LWL-Materials, zum Beispiel in umgekehrter Proportionalität, angepasst werden. Die FBG-Sensoren können in einer Ausgestaltung der Erfindung als ein Endlosgitter ausgestaltet sein, das heißt die Abstände zwischen FBG-Sensoren sind sehr klein und gehen gegen Null. Hierdurch ist eine Laufzeitmessung oder eine Messung verschiedener Reflexionspeaks möglich, die wiederum Rückschlüsse auf Drücke erlauben. Auch eine Frequenzänderung des Gitters, zum Beispiel gechirpte Strukturen, der FBG-Sensoren kann vorteilhaft sein. In einer weiteren Ausgestaltung sind mindestens zwei Messzellenträger in einer Schutzhülle oder einem Mantel integriert, wobei die FBG-Sensoren der Messzellenträger überlappend zueinander angeordnet sind und somit auch eine Laufzeitmessung ermöglicht wird. Die Bragg-Wellenlängen der FBG-Sensoren können in solch einer Ausgestaltung aufeinander abgestimmt sein und beispielsweise überlappen.

Ein Vorteil von FBG-Sensoren ist, dass sie gegenüber elektromagnetischen Störungen unempfindlich sind und durch elektromagnetische Felder keine Induktion erfahren. Auch sind sie für Strahlen weitgehend transparent. Hierdurch sind Druckmessvorrichtungen mit FBG-Sensoren als Messzellen in der Anwendung gut mit anderen diagnostischen oder therapeutischen Apparaten (Magnetresonanztomographen, Röntgenstrahler, Kernspinresonanzspektroskope, Elektronen- / Partikelstrahler und so weiter) kombinierbar, was wiederum die Komplexität der möglichen Diagnostik oder Therapien erstaunlich erweitert.

Bei der Einbringung mehrerer FBG-Sensoren in einen LWL ist vorteilhafterweise darauf zu achten, dass die verwendeten FBG-Sensoren sich jeweils zueinander ausreichend unterscheiden, um bei der Auswertung ein Übersprechen der Reflexionsmaxima der Bragg-Wellenlängen gedehnter oder gestauchter Gitter auf andere eingebrachte FBG-Sensoren zu vermeiden.

Da die zur Verfügung stehenden Lichtfrequenzbänder durch die optischen Eigenschaften der LWL beschränkt sind, können die bevorzugten FBG-Sensoren für die jeweils geplante Anwendung gezielt ökonomisiert eingesetzt werden: In biologischen Systemen sind zunächst nur geringe, gegebenenfalls vernachlässigbare Temperaturdifferenzen zu erwarten. Auch herrscht oft ein hydrostatischer Grunddruck vor, der auf den eingebrachten Teil der Druckmessvorrichtung im Ganzen wirkt. Die klinisch relevanten und vorherrschenden Druckamplituden liegen je nach Anwendungsort beispielsweise im koronaren Herzkreislaufsystem in Höhe der Systolen-Diastolen-Differenz bis circa 150 mmHg (entspricht circa 20 kPa), im Wirbelkanal im Bereich feiner fortgepflanzter Schwingungen durch das HerzKreislaufsystem bis circa 10 mmHg (entspricht circa 1333 Pa). Es können also in den verfügbaren Lichtfrequenzbereichen des jeweiligen LWL-Materials beispielsweise in wirbelsäulen-chirurgischen Anwendungen mehr FBG-Sensoren und mit engeren Differenzen ihrer Gitterabstände (also ihrer jeweiligen resultierenden Bragg-Wellenlängen) zueinander verwendet werden als beispielsweise in koronaren Anwendungen.

Erweiternd kann es vorteilhaft sein, dass der Messzellenträger mindestens einen Marker aus einem Metall oder Übergangsmetall, insbesondere einen röntgenstrahlendichten metallischen Marker aufweist, der in oder an einer Schicht des Messzellenträgers vorliegt, um so eine Position des Messzellenträgers in dem biologischen System durch ein bildgebendes Verfahren sichtbar zu machen. Durch derartige Einbringungen kann die Lage der Druckmessvorrichtung in dem biologischen System bestimmt werden, was beispielsweise von Vorteil sein kann, wenn eine Operation an dem biologischen System durchgeführt oder geplant wird und mittels der Druckmessvorrichtung für die Operation relevante Daten erfasst werden sollen. So können neben den Daten über die Drücke auch die Lage und Position der Druckmessvorrichtung in einem bildgebenden Verfahren ermittelt werden und für den Behandler zwei- oder dreidimensional, bevorzugt farbkodiert, und insbesondere über den zeitlichen Verlauf, dargestellt werden. Gerade wenn die Messungen an biologischen Systemen in verschiedenen Stellungen durchgeführt werden, kann diese Zuordnung von hoher klinischer Bedeutsamkeit sein. Ebenso können im Verlauf der Druckmessvorrichtung eingebrachte röntgendichtere Marker unter RöntgenDurchleuchtung ein Wiederauffinden nach akzidentiellem Abreißen der Druckmessvorrichtung ermöglichen.

Bei klinischen Fragestellungen ist es häufig bedeutsam zu erfahren, ob eine Engstelle im biologischen System eine klinische Relevanz hat. Die Messung von Druckamplituden vor und nach einer fraglichen Engstelle gibt hier wertvolle Hinweise. Im Bereich der Wirbelsäule konnte gezeigt werden, dass Engstellen, die zu einem weitgehenden Verebben der im Wirbelsäulenkanal vorkommenden Schwingungsamplituden oberhalb des hydrostatischen Grunddruckes führen, operationswürdiger sind. Im Bereich der koronaren Intervention sind häufig Engstellen von klinischer Signifikanz, die die systolische Druckwellenausbreitung verändern.

Wegen ihrer besseren Kompatibilität mit MRT-Untersuchungen können als röntgendichte Marker Übergangsmetalle wie Tantal vorteilhaft sein. Alternativ können MRT-Resonatoren in die Druckmessvorrichtung integriert werden, so dass die Druckmessvorrichtung in einer MRT-Untersuchung sichtbar ist. Die Marker können in Form von Schienen, Hülsen oder Netzen an einer Schicht der Druckmessvorrichtung angebracht werden. Die röntgendichten Marker können also auch in einfacher Weise nachträglich mit der Druckmessvorrichtung verbunden werden. Die Marker können jedoch auch in der Druckmessvorrichtung eingebracht vorliegen, indem die Marker beispielsweise als Partikel während der Herstellung den Schichten der Druckmessvorrichtung zugefügt werden. Die röntgendichten Marker können auch derart auf die Druckmessvorrichtung aufgebracht sein, dass sie in Form von Markierungen, wie beispielsweise Striche oder Kreise vorliegen und zur optischen Ablesung der Einschubtiefe der Druckmessvorrichtung in das biologische System herangezogen werden können.

Diesbezüglich kann es vorteilhaft sein, wenn der metallische Marker gleichzeitig als Ummantelung der Referenzmesszelle Verwendung findet, so dass die Referenzmesszelle lediglich den Temperatureinfluss auf das Faser-Bragg-Gitter erfasst und zusätzlich die Ummantelung als Marker für ein bildgebendes Verfahren herangezogen werden kann.

Die Messzellen können in einer Ausführungsform aus preisgünstigen Telekomunikations-LWL hergestellt werden, beispielsweise mit handelsüblichen 80 oder 125 µm Manteldurchmesser.

Der Messzellenträger kann in einer Ausgestaltung der Erfindung einen piezoelektrischer Sensor in der ersten und / oder zweiten Schicht aufweisen. Der Messzellenträger kann ein Kabel, beispielsweise ein Kabel mit Kupferleitern, in seinen Schichten aufweisen, in das ein oder mehrere piezoelektrische Sensoren integriert sind. Die piezoelektrischen Sensoren bestehen vorteilhafterweise aus piezoelektrischen Keramiken (insbesondere modifiziertes Bleizirkonat-Titanat (PZT) oder Bariumtitanat) oder aus einkristallinen Materialien. Als einkristalline Materialien sind Quarz, Turmalin, Galliumphosphat und Aluminiumnitrid bevorzugt. Die bevorzugten piezoelektrischen Sensoren besitzen eine hohe Sensitivität und Langzeitstabilität. Die piezoelektrischen Sensoren sind bevorzugt in Reihe geschaltet und innerhalb des Messzellenträgers, insbesondere des Kabels parallel zu einer Kabel-Achse angeordnet. Vorteilhafterweise kann der piezoelektrische Sensor, der am Auswerteeinheit-fernen Ende des Messzellenträgers angeordnet ist, quer zur Achse angebracht werden, so dass ein Druck auf die Spitze des Messzellenträgers durch den piezoelektrischen Sensor erfassbar ist. Dadurch, dass die piezoelektrischen Sensoren vorzugsweise mit einem elektrischen Leiter geschaltet sind, wächst mit steigender Anzahl der Sensoren auch der Durchmesser des Messzellenträgers. Um den Kathetergesamtdurchmesser gering zu halten, können die piezoelektrischen Sensoren vorzugsweise nacheinander durch ein Bus-System angesteuert werden. Hierbei können digitale Schaltkreise oder einfache Logikbausteine Verwendung finden, um den jeweiligen piezoelektrischen Sensor anzusprechen.

In einer bevorzugten Ausführungsform der Erfindung weist der Messzellenträger mindestens zwei Schichten auf, wobei die Messzellen in einer ersten und / oder einer zweiten Schicht angeordnet sind. Der Messzellenträger, insbesondere der LWL oder das Kabel weisen mehrere Schichten auf. Der LWL besteht aus einem Kernmaterial, einem Mantelmaterial und vorzugweise einer Schutzschicht. Das Kabel ist vorzugsweise aus einem elektrisch leitenden Bereich, einem Mantelbereich und vorzugsweise einer auf den Mantelbereich aufgebrachten, biokompatiblen Schutzschicht aufgebaut. Die biokompatible Schutzschicht kann analog wie bei einem LWL ausgestaltet sein. Es hat sich überraschenderweise herausgestellt, dass die Messzellen in einer ersten und/oder einer zweiten Schicht des Messzellenträgers angeordnet sein können und hierdurch die Sensitivität der Druckmessvorrichtung und das örtliche Auflösungsvermögen der einwirkenden Kräfte verbessert werden kann.

Es konnte gezeigt werden, dass Biegungen oder Temperaturschwankungen des Messzellenträgers Störungen in der Druckmessung durch Messzellen, insbesondere FBG-Sensoren bewirken. Eine Detektion der Biegungen kann somit vorteilhaft sein. Eine Biegung des Messzellenträgers bewirkt gleichzeitig eine Dehnung und Stauchung des Messzellenträgers. In oder um den Mantel eingebrachte Messzellen, insbesondere FBG-Sensoren, können im Falle einer Biegung abhängig von ihrer Lage mit unterschiedlichen reflektierten Bragg-Wellenlängen antworten, das heißt die an der Biegungsinnenseite befindlichen FGB-Sensoren werden insbesondere gestaucht, die an der Außenseite befindlichen gestreckt, was zu zwei unterschiedlichen Reflexionsmaxima führt. Aus der Kenntnis dieses Phänomens lassen sich überraschenderweise Biegungen des Messzellenträgers, insbesondere des LWLs ableiten. So können beispielsweise Druckmesswerte in gebogenen Abschnitten verworfen werden. Es kann jedoch ebenso vorteilhaft sein auch die Drücke in den Biegungen zu erfassen. Beispielsweise kann durch eine Auswertesoftware der Nutzer auf die Störungsgefahr durch Biegung hingewiesen werden, gegebenenfalls kann die Software auch die Druckmesswerte der FBG-Sensoren im Kernbereich durch Bestimmung der Biegung aus dem Mantel rechnerisch korrigieren. Ferner kann es vorteilhaft sein, wenn mithilfe von Dehnungsmesstechnik eine gleichzeitige Messung von longitudinalen Bewegungen und Biegungen in der Druckmessvorrichtung möglich sind, so dass eine Lage- und Formbestimmung der eingebrachten Druckmessvorrichtung erreicht werden kann. In einer besonderen Ausführungsform können zirkulär im LWL-Mantel eingebrachte FBG-Sensoren zur Biegungsmessung und gleichzeitig vorhandene FBG-Sensoren im LWL-Kern zur Druckmessung herangezogen werden. Weiterhin können in einer Ausführungsform aus Gründen der Lichtfrequenzökonomie Splitphänomene der FBG-Sensoren zur Biegungsdetektion genutzt werden. Zusätzlich kann die Richtung der Dehnung bestimmt werden, indem im Mantel bevorzugt zirkulär Felder verschiedener FBG-Sensoren eingebracht werden. Zusammenfassend können also überraschenderweise Position an der Druckmessvorrichtung, Ausmaß und Richtung der jeweiligen Biegung errechnet werden. In einer Ausgestaltung der Erfindung kann an der Spitze der Druckmessvorrichtung, also am Auswerteeinheit-fernen Ende, eine Messzelle angeordnet sein. Diese Messzelle, beispielsweise eine FBG-Sensor oder ein piezoelektrischer Sensor oder auch ein Fabry-Perot-Interferometer kann dazu genutzt werden, um ein Festsitzen der Druckmessvorrichtung zum Beispiel im Gewebe frühzeitig zu erkennen und den Behandler gegebenenfalls durch eine Auswertesoftware zu warnen. Zusätzlich können auch Temperaturartefakte eliminiert werden.

Es kann vorteilhaft sein, wenn der Messzellenträger mindestens einen Arbeitskanal mit einem Eingang und gegebenenfalls Ausgang aufweist. Der Messzellenträger kann einen oder mehrere insbesondere parallel zur Längsachse des Messzellenträgers verlaufende Arbeitskanäle aufweisen. Über die Arbeitskanäle kann Material, beispielsweise Medikamente, in das biologische System eingebracht aber auch diesem entnommen werden. Außerdem können Miniaturgeräte oder optische oder lichtenergieleitende Systeme, sowie Führungsdrähte eingebracht werden oder durch einen oder mehrere an der Messzellenträger-Außenwand angebrachte oder in die Messzellenträger-Außenwand eingebrachte Ballons Dilationen vorgenommen werden. Auch die Einbringung und Platzierung von Stents wäre möglich. Ebenso können durch auch nur vorübergehend eingebrachte Elektroden Ableitungen, Stimulationen oder Koagulationen in dem biologischen System vorgenommen werden. Die Elektroden und deren Zuleitungen können ebenfalls durch leitende Übergangsmetalle ausgeführt sein. Auch die Applikation von Medikamenten kann vorteilhafterweise über die Druckmessvorrichtung, insbesondere durch einen Arbeitskanal erfolgen. Auch ist eine gezielte endoluminale Varizenverödung durch die Druckmessvorrichtung, insbesondere durch einen oder mehrere Arbeitskanäle, möglich. Die Arbeitskanäle können an der Spitze der Druckmessvorrichtung oder im Verlauf der Druckmessvorrichtung seitlich ihre jeweiligen Ausgänge haben. Elektroden können zur Vermeidung von Störungen beispielsweise bei MRT-Untersuchungen erst kurz vor der Verwendung in die Druckmessvorrichtung eingeführt und dann gegebenenfalls wieder entnommen werden.

Ebenso können in einer Ausführungsform eine oder mehrere Elektroden permanent am Druckmessgerät angebracht sein. In einer weiteren Ausführungsform hat der Arbeitskanal mehrere Ausgangsöffnungen, zum Beispiel um hierüber eingebrachte Flüssigkeiten gleichmäßiger im Organismus zu verteilen oder um bei einem Anliegen an Gewebestrukturen alternative Austrittsmöglichkeiten vorzuhalten.

In einer bevorzugten Ausgestaltung der Erfindung ist der Messzellenträger am Auswerteeinheit-nahen Ende über einen Koppler, einen Konnektor oder Zirkulator mit einer Lichtquelle und einem optischen Sensor verbunden. Die Ansteuerung der FBG-Sensoren erfolgt bevorzugt über gängige Lichtquellen, wie zum Beispiel, jeweils fasergekoppelt, durchstimmbare schmalbandige Laser oder breitbandige Lichtquellen, wie Superluminescent Light Emitting Diodes (SLED). Die Druckmessvorrichtung kann in einer Ausgestaltung außerdem über einen sterilisierbaren optischen Steckanschluss, zum Beispiel einen modifizierter FC/APC-Stecker (Englisch für _{"}angle physical contact"), verfügen. Die durchstimmbare schmalbandige Lichtquelle erfordert ein Abstimmen der Lichtquelle auf die erwartete Reflexionswellenlänge des jeweiligen FBG-Sensors sowie eine, bevorzugt durchlaufende, Abstimmung in einem schmalen Bereich über und / oder unterhalb dieser Wellenlänge, um eine Veränderung des Reflexionsmaximums des jeweiligen FBG-Sensors zu messen (zum Beispiel als Scanning). Das heißt, es ist vorteilhafterweise möglich, jeweils einzelne FBG-Sensoren mit dem entsprechenden schmalen Wellenlängenbereich anzusprechen. Ebenso ist es vorteilhafterweise möglich, mehrere, verschiedene, FBG-Sensoren gleichzeitig mit Licht eines breiteren Wellenlängenbereiches anzusteuern.

Die Auswertung der durch die FBG-Sensoren reflektierten Wellenlängen kann bevorzugt mittels bekannter optischer Sensoren erfolgen, wobei je nach eingesetzter Lichtquelle ein Fotosensor oder ein System aus Lichtfrequenztrenner und Sensor (beispielsweise Monochromator und Charge-coupled Device(CCD)-Zeile(n)) Anwendung finden kann. Die Lichtquelle und der Sensor sind insbesondere mit einem Koppler oder einem Zirkulator und einem Konnektor mit dem LWL verbunden. Die Erfassung der Daten und die Auswertung kann über eine Datenverarbeitungseinheit erfolgen, wobei anhand der Reflexions-Lichtwellenlängen und / oder Peak-Messungen und durch eine Zuordnung der Messwerte zu den entsprechenden Messzellen, die resultierenden Druckverhältnisse anhand der Veränderungen der Messwerte, insbesondere in Bezug zu einer Normierungsgröße berechnet werden. Die Datenverarbeitungseinheit erhält vorteilhafterweise kontinuierlich oder diskontinuierlich Messergebnisse, beispielsweise mit 50 Hz pro Messzelle, über den jeweiligen Gitter-Zustand der FBG-Sensoren in der Messzelle. Hierdurch sind Rückschlüsse auf die Druckverhältnisse relativ zur Lage des Messzellenträgers im biologischen System möglich, sowie ergänzend Aussagen zur Temperatur möglich. Es ist vorteilhafterweise auch ein Druckmesssystem vorgesehen, umfassend einen Messzellenträger mit Messzellen, eine Lichtquelle, einen optischen Sensor, einen optischen Koppler oder Zirkulator und eine Datenverarbeitungseinheit, gegebenenfalls ein Datenspeicher, gegebenenfalls Energie- und Kommunikationsversorgung. Ferner kann mittels einer entsprechenden Hard- und / oder Software ein Interface zwischen einer gemessenen Herzfrequenz und den durch die Druckmessvorrichtung gemessenen Drücken eine Korrelation hergestellt werden, wobei die Druckmessvorrichtung EKG- oder Pulswellen getriggert sein kann. Ebenso können vorteilhafterweise auch andere Dynamiken des Organismus zum Triggern verwendet werden, zum Beispiel Atmung oder Bewegungen. Die von der Druckmessvorrichtung erfassten Messungen können periodisch oder kontinuierlich an ein außerhalb des biologischen Systems angeordneten Gerät übermittelt werden, wobei in Abhängigkeit der Ausgestaltung der Druckmessvorrichtung Drücke, Temperaturen, räumliche Veränderungen, Schwingungen, Vibrationen und / oder Beschädigung / Abriss / Zerstörung der Druckmessvorrichtung gemessen werden können.

Mit steigender Abtastfrequenz der Messzellen können die Druckverläufe insbesondere im zeitlichen Verlauf mit ihren Amplitudenverläufen zunehmend besser dargestellt werden. Gegebenenfalls können Amplituden durch Interpolation für den Untersucher besser lesbar dargestellt und damit geringere Abtastfrequenzen zu einem gewissen Grad kompensiert werden. Die Beurteilung der Amplitudenverläufe kann dem Untersucher weitere Rückschlüsse auf die Funktionalität des untersuchten biologischen Systems geben.

In einer bevorzugten Ausgestaltung der Erfindung ist eine Messzelle, insbesondere ein FBG-Sensor oder ein piezoelektrischer Sensor, in der Spitze des ersten Endes des Messzellenträgers integriert, wobei der Sensor quer zur Längsachse des Messzellenträgers oder in axialer Richtung zur Längsachse vorliegt. Der Sensor kann insbesondere dazu verwendet werden, einen Kontakt zwischen der Druckmessvorrichtung und zum Beispiel einer Begrenzung oder Begrenzungsfläche eines biologischen Systems festzustellen, was wiederum für die Führung der Druckmessvorrichtung in dem biologischen System erforderlich sein kann.

Ferner ist es bevorzugt, je nach Anwendungsgebiet der Druckmessvorrichtung die Messzellen, insbesondere die FBG-Sensoren, in dem Messzellenträger in verschiedenen Gitterabständen anzuordnen, wobei diese Messzellen an die in einem biologischen System vorliegenden unterschiedlichen Druckverhältnisse angepasst sind. Die Anpassungen können neben variierenden Gitterabständen auch Abstände zwischen mehreren FBG-Sensoren umfassen.

Vorteilhafterweise können mindestens zwei Messzellenträger in der aus einem biokompatiblen Material bestehenden Schutzschicht angeordnet sein, wobei es auch bevorzugt ist, dass mindestens drei Messzellenträger in der aus einem biokompatiblen Material bestehenden Schutzschicht angeordnet sind. Die Schutzschicht kann beispielsweise eine Kunststoffhülle sein, wobei auch andere Materialien wie Metall verwendet werden können. Somit kann die Druckmessvorrichtung derart ausgestaltet sein, dass mehrere Messzellenträger von einer Schutzschicht oder -hülle umgeben sind. Die Messzellenträger, insbesondere die Lichtwellenleiter, können gemeinsam extrudiert werden. Dazu liegen die, das heißt mehrere, Lichtwellenleiter bevorzugt in einer Pinole, wobei aber auch eine Fertigung im Spritzguss des sensorisch aktiven Teils des Lichtwellenleiters, das heißt insbesondere des Bereichs, in dem eine Messzelle angeordnet ist, möglich ist. Auch ist durch dieses Herstellungsverfahren ein Hinzufügen oder Einbringen eines Arbeitskanals leicht durchführbar. Ferner kann es vorteilhaft sein, die Druckmessvorrichtung koaxial aufzubauen, so dass mehrere Messzellenträger um einen Arbeitskanal herum angeordnet werden und der Arbeitskanal mit Versteifungsmitteln, beispielsweise mit starren Kathetern variabel und in Abhängigkeit von der jeweiligen Anwendung befüllt werden und so die Steifigkeit der Druckmessvorrichtung angepasst werden kann. Die FBG-Sensoren können parallel, helikal, spiral, einfach, mehrfach oder dreifach zu einem, beziehungsweise um einen solchen Arbeitskanal ausgerichtet sein.

Eine weitere vorteilhafte Ausführungsform besteht aus mindestens drei, koaxial um eine innere, mittige Struktur in etwa gleichen Abständen angeordnete Messzellenträger, zum Beispiel bei 0°, 120° und 240°. Diese Struktur kann ein weiterer Messzellenträger und / oder ein Arbeitskanal sein. Die koaxial angeordneten LWL können hier bevorzugt der Form-/Biegungsmessung der Druckvorrichtung dienen, aber ebenso auch der Druckmessung, hingegen die innere, mittige Leitung als Messzellenträger ausgeführt sein und der Druckmessung oder / und zur Temperaturreferenz dienen. Ebenso kann in einer weiteren Ausführungsform der innere Messzellenträger insbesondere als LWL oder auch als elektrischer Leiter ausgeführt sein und auch oder nur der Versorgung eines Tip-Sensors oder eines Fabry-Perot-Interferometers dienen.

Diesbezüglich ist es bevorzugt, wenn die Messzellenträger in Längsrichtung der Schutzschicht versetzt zueinander angeordnet sind. Die Schutzschicht weist eine Längsrichtung auf, wobei die Schutzschicht im Sinne der Erfindung auch als Schutzhülle bezeichnet werden kann. Die Messzellenträger, das heißt, insbesondere die LWL sind in der Schutzhülle exzentrisch zueinander angeordnet. Insbesondere sind die in einem LWL vorliegenden FBG-Sensoren zu FBG-Sensoren eines zweiten und gegebenenfalls mindestens eines weiteren LWLs versetzt angeordnet. Es hat sich als besonders vorteilhaft herausgestellt, wenn die Messzellenträger zueinander um 1/n eines Pitches eines FBG-Sensors versetzt zueinander angeordnet sind und n die Anzahl der Messzellenträger in der Schutzschicht ist. Ein Pitch ist insbesondere der Abstand von der Mitte eines FBG-Sensorfeldes bis zur Mitte des nächstliegenden FBG-Sensorfeldes innerhalb des gleichen Messzellenträgers, wobei ein FBG-Sensorfeld ein gleiches Gitter mit gleicher Gitterweite ist. Dies ermöglicht überraschenderweise eine Erkennung und Separierung von biegungs-, dehnungs- und radialkraftbedingten Veränderungen der Reflexionswellenlänge eines FBG-Sensors und resultiert sowohl in einer Verbesserung der Ortsauflösung als auch in einer, über die mit FBG-Sensoren bestückten Teile des Druckmessvorrichtung hin, örtlich-unterbrechungsfreien Sensorik der FBG-Sensoren, als es eine Anordnung der FBG-Sensoren auf jeweils gleicher Höhe über verschiedene Messzellenträger allein bietet. Es können somit Drücke entlang des Messzellenträgers im Wesentlichen unterbrechungsfrei erfasst werden.

Um eine einfache Führung der Druckmessvorrichtung in beispielsweise einem biologischen System zu erreichen, kann die Druckmessvorrichtung, zum Beispiel im Arbeitskanal oder der Schutzhülle, ein Versteifungsmittel aufweisen. Hierbei kann es sich um ein drahtförmiges Mittel aus Kunststoff handeln, das beispielsweise mittig in der Druckmessvorrichtung, insbesondere der Schutzhülle angeordnet ist. Als Kunststoff für das Versteifungsmittel können beispielsweise Polymeren oder aromatische Polyamide verwendet werden. Das Versteifungsmittel kann auch gewebeförmig ausgestaltet sein. Hier bietet sich besonders ein Gewebe aus den zuvor genannten bevorzugten Kunststoffen an. Das Versteifungsmittel ist insbesondere dann von Vorteil, wenn mehrere Messzellenträger in der Druckmessvorrichtung, insbesondere der Schutzhülle vorliegen.

Um Verformungen beziehungsweise auf die Druckmessvorrichtung wirkende Kräfte an der Spitze der Druckmessvorrichtung zu bestimmen, ist in dem Auswerteeinheit-fernen Ende ein Interferometer angeordnet, wobei im Falle des Vorhandenseins einer atraumatischen Spitze, das Interferometer auch in der atraumatischen Spitze angeordnet sein kann. Das Interferometer ist ein Fabry-Perot-Interferometer oder kann in einer weiteren Ausgestaltung ein Michelson-Interferometer sein, wobei auch sonstige Vielstrahlinterferometer verwendbar sind. Das Fabry-Perot-Interferometer weist mindestens einen optischen Resonator auf, der aus zwei teildurchlässigen Spiegeln gebildet ist. In der Spitze oder dem Faserende der Druckmessvorrichtung ist zudem ein Reflektor, das heißt, eine Licht-reflektierende Oberfläche angeordnet. Es kann auch bevorzugt sein, den Reflektor als Teil des Fabry-Perot-Interferometers auszugestalten. Der Fabry-Perot-Interferometer kann als Mikrosystem (Micro-Electro-Mechanical-System - "MEMS") gefertigt sein, das heißt, als ein miniaturisiertes Gerät, dessen Komponenten kleinste Abmessungen (im Mikrometerbereich) haben und als System zusammenwirken. Auch ist eine Herstellung des Interferometers durch ein Aufdampfen von dünnen Schichten mit einem Luftspalt an der Spitze mithilfe einer Dünnschicht-Technologie möglich. Der Fabry-Perot-Interferometer bildet somit zusätzlich zu den im Messzellenträger angeordneten Messzellen eine weitere Messzelle, die im Wesentlichen an der Spitze (dem Auswerteeinheit-fernen Ende) oder dem Faserende angeordnet ist. In einer Ausgestaltung bildet der Fabry-Perot-Interferometer einen Drucksensor, welcher im gleichen Wellenlängenfenster auswertbar ist wie die FBG-Sensoren. In der Spitze oder dem Faserende befindet sich ein, beispielsweise in Mikrosystem-Technologie gefertigtes, Fabry-Perot-Interferometer, welches sich aus mindestens einem Ende eines Messzellenträgers, insbesondere eines LWLs (Auswerteeinheit-fernes Ende) sowie mindestens einer, durch Druckeinwirkung und daraus folgender Verformung der Spitze, deformierbaren verspiegelten Membran zusammensetzt. Am LWL-Ende, also dem Auswerteeinheit-fernen Ende der Druckmessvorrichtung, können teilreflektierende Spiegel angeordnet sein, deren Transmissionsspektrum bevorzugt schmale Transmissions-Maxima für Wellenlängen zeigt, welche Resonanzbedingungen erfüllen, während andere Spektralbereiche in der Transmission nahezu vollständig ausgelöscht werden. Dies geschieht durch konstruktive oder destruktive Interferenz der Teilstrahlen. Dadurch, dass die verspiegelte Membran deformierbar ausgestaltet ist, bewirkt eine einwirkende Kraft (beispielsweise ein Druck) eine Änderung des Transmissionsspektrums und folglich der reflektierten Strahlen, so dass auch geringe Druckänderungen an der Spitze oder dem Faserende messbar sind. Dies ist insbesondere beim Einführen der Druckmessvorrichtung in ein biologisches System und bei dem Durchführen der Druckmessvorrichtung durch dieses vorteilhaft. Zudem ist das Interferometer im Wesentlichen resistent gegen Temperatureinflüsse, das heißt, mithilfe des Interferometers kann ein Druck unabhängig von der Temperatur gemessen werden. Das Interferometer kann somit zum Kalibrieren der Druckmessvorrichtung auf Temperaturartefakte sowie zum Messen von anderen Druckbereichen und anderen Druckauflösungen, gegebenenfalls auch in anderen Messungsfrequenzen, verwendet werden.

Das Interferometer kann zur Kalibrierung der Druckmessvorrichtung genutzt werden, da das Interferometer eine weit geringere Beeinflussung durch Temperatur besitzt, als die FBG-Sensoren. Zudem können durch das Interferometer an der Spitze der Druckmessvorrichtung andere, zum Beispiel feinere oder gröbere, Druckbereiche gemessen werden. Dies kann zur Gewebekontaktdetektion sowie zur Messung absoluter Druckwerte innerhalb des biologischen Systems genutzt werden.

Neben der Druckmessung können die FBG-Sensoren in der Druckmessvorrichtung auch zur Lage- und Formfeststellung verwendet werden, da mithilfe der gemessenen FBG-Daten auf die Lage und Form der Druckmessvorrichtung rückgeschlossen werden kann.

Der Messzellenträger kann in ein biologisches System eingebracht und an einer Messstelle positioniert werden. An der ersten Messstelle kann ein erstes Messergebnis erfasst werden. Dadurch, dass mit dem Verfahren ein Messabstand messbar ist, können zwei Messergebnisse in räumlicher oder zeitlicher Abhängigkeit bestimmt werden Durch eine Lageänderung des Messzellenträgers kann vorteilhafterweise das zweite Messergebnis an einer zu der ersten Messstelle beabstandeten Messstelle erfasst werden. Hierdurch ist es überraschenderweise möglich, zwei Messergebnisse, insbesondere Drücke in Relation zueinander zu erfassen. Einer der beiden gemessenen Drücke kann beispielsweise als lokaler Referenzwert im jeweiligen biologischen System oder im jeweiligen Abschnitt des biologischen Systems herangezogen werden.

Außerdem ist es vorteilhafterweise möglich, ohne Lageänderung an der ersten Messstelle ein zeitliches Messergebnis zu erfassen, wobei ein zweites Messergebnis an der ersten Messstelle an einem dem ersten Messergebnis zeitlich nachfolgenden Zeitpunkt erfasst wird. Hierdurch können an einer Messstelle Drücke über einen zeitlichen Verlauf gemessen werden. Durch ein bevorzugt automatisiertes Verschieben des Messzellenträgers der Länge nach oder insbesondere der Messzellen innerhalb eines weiteren Mantels um die Länge des Abstandes zweier Messzellen, können räumlich durchgehend Umgebungsdruckwerte erfasst werden.

Mit dem bevorzugten Verfahren kann vorteilhafterweise eine Erfassung von Messergebnissen über einen zeitlichen Verlauf durchgeführt werden, wobei zusätzlich eine Lageveränderungen des Messzellenträgers oder des biologischen Systems (insbesondere einem Patienten) erfolgen kann (beispielsweise sitzend / liegend / stehend, Bauch- / Rückenlage oder festgelegte Bewegungen und Belastungen).

Ferner können durch eine Kopplung der Messdaten mit Bilddaten aus bildgebenden Verfahren (zum einen Lageerfassung des Messzellenträgers, zum anderen morphologische Erfassung des biologischen Systems) die Umgebungsdruckdaten, insbesondere auch die Differenzen in Druckamplituden, grafisch, beispielsweise farblich, dargestellt werden. In Verbindung mit den Grauwertinformationen aus der Bildgebung kann hier für den Behandler überraschenderweise einfach eine Ablesung ermöglicht werden. Durch die Einbringung von röntgendichteren Materialien an definierten Punkten der Druckmessvorrichtung kann zudem eine Positionierung und Lage- und Ausdehnungsbestimmung erfolgen. Mit Hilfe einer Software, insbesondere mithilfe eines Verfahrens zur Bilderzeugung kann die Position und / oder Form der Druckmessvorrichtung zusammen mit weiteren Messdaten zu einem zwei- oder dreidimensionalen Bild zusammengeführt werden; gegebenenfalls auch über den zeitlichen Verlauf. Zur Bilderzeugung können die Daten der Druckmessvorrichtung, insbesondere des Druckmesssystems in Kombination mit weiteren Daten, beispielsweise aus bildgebenden Verfahren mittels einer Datenverarbeitungseinheit ausgewertet werden und grafisch auf einer Ausgabeeinheit, beispielsweise einem Monitor angezeigt werden. Es ist ferner eine farbliche Bildkodierung der mit der Druckvorrichtung erfassten Amplitudendifferenzen und Druckverhältnisse bevorzugt. Auch können die Daten der Druckmessvorrichtung gegebenenfalls akustisch dargestellt werden.

Die erfindungsgemäße Druckmessvorrichtung sowie das Verfahren können in unterschiedlichen biologischen Systemen zum Einsatz kommen, wobei keine wesentlichen Modifizierungen der Druckmessvorrichtung vorgenommen werden müssen. Außerdem ist die Druckmessvorrichtung sterilisierbar, da die Messzellen und der Messzellenträger die hohen Temperaturen der üblichen Sterilisatoren beziehungsweise die Bedingungen der chemischen und / oder physikalischen Sterilisation unbeschadet aushalten und eine volle Funktionsfähigkeit beibehalten. Dies ist gegenüber den bekannten Druckmessvorrichtungen als besonders vorteilhaft anzusehen, da diese oftmals als Einwegartikel ausgestaltet sind und hierdurch hohe Kosten verursachen. Die erfindungsgemäße Druckmessvorrichtung kann dahingegen einfach und schnell mittels bekannter Sterilisationsverfahren sterilisiert werden. Es kann jedoch für einzelne Anwendungen oder biologische Systeme vorteilhaft sein, wenn die Druckmessvorrichtung als Einwegartikel ausgestaltet ist. Um eine sichere und effektive Sterilisation der Druckmessvorrichtung zu erreichen, kann die Druckmessvorrichtung in dem Sterilisator mit einer Halterungsvorrichtung verbunden sein. Nach einer erfolgreichen Sterilisation kann die Druckmessvorrichtung, beispielsweise aufgerollt, in einem Aufbewahrungs- oder Transportmittel bis zur nächsten Druckmessung aufbewahrt werden. Ein solches Mittel kann beispielsweise ein Röhrchen sein, aus dem sich die Druckmessvorrichtung herausschieben und wieder zurückschieben lässt.

Vorteilhafterweise kann das Verfahren, beziehungsweise die Druckmessvorrichtung zur Auswertung der Differenzen in den biologisch vorkommenden (insbesondere pulsabhängigen) Druckamplituden an den verschiedenen Messstellen der Druckmessvorrichtung verwendet werden. Dieses so gemessene Zwischenergebnis kann wiederum Rückschlüsse auf Lage(n) und klinische Bedeutsamkeit(en), beispielsweise lumbaler Spinalstenosen oder Herzkranzgefäßstenosen, zulassen.

Es hat sich überraschenderweise durch Versuche herausgestellt, dass unter anderem die folgenden Verwendungen der Druckmessvorrichtung und des Verfahrens möglich sind: Kardiologie / Kardiochirurgie (zum Beispiel Druckmessung in Koronargefäßen, intraaurikuläre Druckmessung, intravaskulärer Ultraschall) Messung des Blutdrucks bei liegenden Sensoren (absolut und im Gefäßverlauf, zeitlich und örtlich aufgelöst); Phlebologie / Angiologie (zum Beispiel invasive intravenöse / intraarterielle Druckmessung und Medikamentenapplikation, Endosonographie, Bougierung und Ballondilatation) / Gefäßchirurgie (zum Beispiel intraoperative Druckmessung in der Karotis-Chirurgie, Endosonographie); Gastroenterologie (zum Beispiel Druckmessung im oberen Gastrointestinaltrakt, zum Beispiel bei Dysphagie oder sekundärer Störungen im Bewegungsablauf des Ösophagus, Diagnosestellung bei Refluxösophagitis, Abklärung von Schmerzen im Brustraum, zum Beispiel auch nichtkardialer Natur, Endosonographie, Bougierung und Ballondilatation) / Viszeralchirurgie (zum Beispiel bei Refluxösophagitis, jeweils prä- und postoperativ, sowie bei abdominellem Kompartmentsyndrom nach Aortachirurgie); Proktologie (zum Beispiel Druckmessung im unteren Gastrointestinaltrakt, abdominelle Druckmessung im Rektum, Endosonographie, Bougierung und Ballondilatation); Endokrinologie / Diabetologie (zum Beispiel Diabetes-Diagnostik, Endosonographie, Bougierung und Ballondilatation); Pneumologie (zum Beispiel Druckmessung bei Lungenemphysem) / Thoraxchirurgie (zum Beispiel Druckmessung im Pulmonalkreislauf, zum Beispiel bei Pulmonalarterienstenose, Endosonographie, Bougierung und Ballondilatation); Nephrologie (zum Beispiel intratubuläre, -vesikale und -uretrale Druckmessung, Endosonographie, Bougierung und Ballondilatation, Dialyse-Indikationsstellung) / Urologie (zum Beispiel Harninkontinenz, postoperative Stress- / Urge-Inkontinenz, Detrusorinstabilität, neurogene Blasenfunktionsstörungen, subvesikalen Obstruktion, Enuresis diurna et nocturna); Gynäkologie / Geburtshilfe (zum Beispiel Endosonographie, Bougierung und Ballondilatation, zum Beispiel zur Fertilitätsdiagnostik / -therapie, intrauterine Druckmessung, zum Beispiel zur Diagnostik der Wehenaktivität und / oder simultane kontinuierliche Amnioninfusion); Neurologie / Psychiatrie / Neurochirurgie (zum Beispiel intrakranielle Diagnostik und Therapie); Schmerzmedizin / Wirbelsäulenchirurgie (zum Beispiel Diagnostik / Therapie von Spinalstenosen / Bandscheibenveränderungen); Rheumatologie (zum Beispiel Endosonographie, Bougierung und Ballondilatation, zum Beispiel bei rheumatischen Erkrankungen des oberen / unteren Gastrointestinaltraktes); Orthopädie (zum Beispiel intraartikuläre Druckmessung im Hüftgelenk, funktionelles Kompartmentsyndrom der Muskellogen, intraossäre Druckmessung); Traumatologie (zum Beispiel traumatisches Kompartmentsyndrom der Muskellogen); Pädiatrie / Kinderchirurgie (zum Beispiel Abklärung von Harnblasenfunktionsstörungen, zum Beispiel bei Mb. Hirschsprung, primäre Störungen im Bewegungsablauf des Ösophagus zum Beispiel bei Achalasie, diffusem Ösophagusspasmus oder Nussknacker-Ösophagus, Endosonographie, Bougierung und Ballondilatation); Hals-Nasen-Ohren-Heilkunde (zum Beispiel Tinnitus- Diagnostik, Diagnostik bei Störungen der Tuba auditiva, Endosonographie); Mund-Kiefer-Gesichtschirurgie (zum Beispiel intrakranielle Druckmessung bei Nervus opticus Laesion); Opthalmologie (zum Beispiel intraokuläre Druckmessung); Hämatologie / Onkologie (zum Beispiel Endosonographie, zum Beispiel vor Feinnadelbiopsie); Anästhesie (zum Beispiel Neuromonitoring, invasive intravenöse / -arterielle Druckmessung und Medikamentenapplikation, intrapulmonale / -tracheale / -pleurale Druckmessung); Radiologie (zum Beispiel Diagnostik der portalen Hypertonie, interventionelle Radiologie); Kontinuierliche zerebrale Druckmessung in einem Spitz Holter Ventil); kontinuierliche / diskontinuierliche Blutdruckmessung, Messung in verschiedenen Gefäßabschnitten (auch intracerebral); kontinuierliche / diskontinuierliche Inkontinenzdiagnostik (urologisch / proktologisch); Darmfunktionsdiagnostik (Peristaltikstärke, Frequenz, Laufzeit), Vorliegen eines Reizdarms; Gynäkologische Überwachung von beispielsweise Uterus, Ovar, Fertilität, Kind; Spannungs- und Schwingungs- und Deformierungsmessung an Implantaten (Platten und Nägel an frakturierten / osteotomierten Knochen) und Endoprothesen; Funktionsdiagnostik, Beurteilung der Konsolidierung, Feedback für Belastungs- und Bewegungssteuerung, Temperatur Abrieb und Reibungskontrolle; Einbau der Druckmessvorrichtung in Orthesen / Bandagen / Einlagen / Korsette zur Spannungs- und Bewegungsmessung und Dokumentation; Steuerung von Exo-Prothesen im Schaft oder im Kunstgelenk zur Stabilisierung einer intelligenten Sensorsteuerung; Kopplung von Endo- und Exo-Prothesen, bei Erkranken oder Gelähmten mithilfe der Druckmessvorrichtung; Einbau der Druckmessvorrichtung in Protektoren (Sport / Forschung) oder Spezialkleidung; Verwendung der Druckmessvorrichtung für ein "Artificial Intelligence Interface", so dass Körperbewegungen in Form von Druck, Berührung, Vibration, Lage und / oder Temperatur übertragen werden und für Roboter / Maschinen nutzbar sind. Auch eine Verwendung der Druckmessvorrichtung als Implantat ist möglich. Hierbei kann beispielsweise ein Teil der Druckmessvorrichtung oder die vollständige Druckmessvorrichtung in Kombination mit einem Datenaufnahmesystem, Energieversorgung und gegebenenfalls einem Sende- und Empfangsmittel als Implantat in ein biologisches System integriert sein, so dass beispielsweise eine Strukturüberwachung von Prothesen über einen Zeitverlauf durchführbar ist. Auch ist mit einer solchen implantierten Druckmessvorrichtung eine kontinuierliche Messung zum Beispiel von Blutdruck und Druck in den Herzkammern möglich, wodurch eine Langzeitblutdruckmessung erreicht werden kann.

Auch kann durch die kontinuierliche Auswertung der im zeitlichen und örtlichen Zusammenhang aufgezeichneten Druckkurven einer natürlichen Druckwelle oder einer Druckwelle, die durch eine geeignete Vorrichtung am Katheter erzeugt wird, ein Rückschluss auf die Umgebungsgewebe gezogen werden. Dazu können die Kennparameter des Druckverlaufes und / oder die örtlichen Reflexionen der Druckwelle oder Druckwellen registriert und ausgewertet werden, so dass daraus die Eigenschaften der Wandung als Gesamtheit (zum Beispiel Verkalkung, Stenosen, Spangen- und Narbenbildung) als auch in der Tiefenausdehnung (Intima- und Plaquequantität- und -qualität, Dicke der Gefäßmittelwand- Media), ähnlich wie bei einer Ultraschallmessung ermittelt werden können. In einer weiteren Ausgestaltung können, beispielsweise durch die um piezoelektrischen-Sensoren ergänzte Druckmessvorrichtung, eine oder mehrere Druckimpulse in den Organismus gesendet werden und die Messergebnisse der Messzellen der im Organismus reflektierten Impulse ausgewertet werden.

Im Folgenden soll eine bevorzugte Verwendung der Druckmessvorrichtung anhand eines Beispiels illustriert werden: Es soll eine Druckdifferenz an einer fraglichen Engstelle in einem Koronargefäß eines Menschen gemessen werden. Zur Einführung der Druckmessvorrichtung in den Blutkreislauf kann eine Einstichstelle vorteilhafterweise lokal betäubt werden. Inwiefern eine Narkose angewendet werden soll, hängt jeweils von der Art und dem Ort der Druckmessung ab. Es kann bevorzugt sein, die Druckmessvorrichtung über eine Schleuse in den menschlichen Organismus einzuführen, so dass ein Wechsel der Druckmessvorrichtung möglich ist, falls diese beschädigt wird oder falls andere Geräte zusätzlich zum Einsatz kommen sollen. Die Schleuse dient insbesondere als flexible Führungsschiene, durch deren Inneres die Druckmessvorrichtung gleiten kann, wobei gleichzeitig die Einstichstelle abgedichtet wird. Nun wird die Druckmessvorrichtung eingeführt. Die Spitze besteht aus weichem, abgerundetem Material (beispielsweise einem Kunststoff), so dass die Gefahr der Beschädigung des Inneren der Blutgefäße möglichst gering ist.

Da die Druckmessvorrichtung insbesondere ein LWL, insbesondere ein optischer oder elektrischer Leiter ist, zum Beispiel ein Kabel ist und bevorzugt einen metallischen Marker aufweist, ist die Druckmessvorrichtung in diesen Ausführungsformen gut bei einer Durchleuchtung mit Röntgenstrahlen zu sehen. So kann ein Kardiologe die Position der Druckmessvorrichtung im Menschen, dem Patienten sehr gut beobachten, wenn er die Druckmessvorrichtung durch das Blutgefäßsystem zum Herzen oder zu den Herzkranzgefäßen hin vorschiebt. Vorteilhafterweise weist der Messzellenträger zwar eine radiale Flexibilität auf, aber ist in der Länge konstant ausgestaltet. Das heißt, die Druckmessvorrichtung wird bei einer Einwirkung einer Schubkraft, wie beispielsweise das Einschieben in das Blutgefäß, nicht nennenswert gestaucht. Da die Herzkranzgefäße selbst jedoch nur schwer erkennbar sind, kann es notwendig werden, dass der Kardiologe örtliche Gefäße durch Gabe eines Kontrastmittels kurzzeitig sichtbar macht. Das Kontrastmittel kann vorteilhafterweise über einen Arbeitskanal der Druckmessvorrichtung eingebracht werden. Zusätzlich kann durch eine im Stand der Technik befindliche elektrokardiographische Kopplung des biologischen Systems mit dem Durchleuchtungssystem das Herz mit seinen Gefäßen jeweils in der gleichen funktionellen Position (Systole / Diastole) abgebildet werden.

Es kann bevorzugt sein, dass durch einen Arbeitskanal der Druckmessvorrichtung ein Führungsdraht eingebracht wird, der bei der Führung der Druckmessvorrichtung durch die Gefäße unterstützend tätig ist. Hierzu kann die Spitze der Druckmessvorrichtung insbesondere gebogen sein. Der Führungsdraht hält die gebogene Spitze der Druckmessvorrichtung bevorzugt gerade, so lange sich der Draht im Inneren des Arbeitskanals befindet.

Wird der Führungsdraht stückweise durch den Kardiologen herausgezogen, nimmt die Spitze der Druckmessvorrichtung ihre bevorzugte beispielsweise gebogene, Form an. Durch wiederholtes Vorschieben und Zurückziehen von Draht und Druckmessvorrichtung kann der Kardiologe gezielt "abbiegen" und sich an die gewünschte Stelle vorarbeiten, was bei der Untersuchung der Herzkranzgefäße besonders interessant ist - schließlich muss hierbei die richtige Abzweigung "getroffen" werden, um weiter vorankommen zu können. Je nach anvisierter Stelle werden unterschiedlich gebogene Formen verwendet. Ist der Kardiologe an der Stelle angekommen, wo die Stenose vermutet wird, können Druckmessungen vorgenommen werden, die Indikation gestellt werden und es kann anschließend gegebenenfalls interveniert werden.

Die Messzellen ändern in Abhängigkeit der auf den Messzellenträger einwirkenden Kräfte ihr Messzellverhalten, was wiederum in Verbindung mit einer Datenverarbeitungsanlage in Druckmesswerte umgerechnet werden kann. Hierdurch kann der Druck in einem Gefäß bestimmt werden sowie Veränderungen von Druckamplituden erfasst werden, wie sie beispielsweise durch klinisch bedeutsame Engstellen verursacht werden. Die Druckmessvorrichtung kann den Druck an einer Messstelle messen, wobei die Druckmessvorrichtung dann an eine weitere Messstelle weitergeführt werden kann, so dass an zwei Messstellen zwei Drücke erfasst werden. Die gemessenen Drücke können in Beziehung zueinander gesetzt werden, um beispielsweise einen auf einer Funktionsstörung beruhenden Druckunterschied festzustellen.

Die Druckmessvorrichtung kann in räumlicher Abhängigkeit Druckmessungen vornehmen. Es kann jedoch auch bevorzugt sein, in zeitlicher Abhängigkeit Druckmessungen durchzuführen, nämlich dann, wenn mehrere Drücke an einer Messstelle zeitlich nacheinander erfasst werden. Hierdurch kann ein zeitlicher Verlauf des Druckes an einer Messstelle bestimmt werden. Die Messung des Druckes mit der Druckmessvorrichtung kann somit auch mit nur einer Messzelle erfolgen, wobei es bevorzugt ist, dass die Druckmessvorrichtung mindestens zwei Messzellen aufweist und eine der beiden Messzellen als Referenzmesszelle zur Messung des Temperatureinflusses auf die Messzelle ausgestaltet ist. Die eigentliche Druckmessung erfolgt sodann nur mit einer Messzelle, insbesondere einem FBG-Sensor. Die Druckmessvorrichtung vorzugsweise mit FBG-Sensoren kann über eine Messstrecke von mehreren bis vielen Zentimetern erfolgen, so dass eine Stenose (Fluss- / Druckwert) in Richtung und Ausdehnung lokalisiert werden kann. Über einen Arbeitskanal der Druckmessvorrichtung kann bevorzugt ein Ballon mit oder ohne Stent dort unter bevorzugter Zuhilfenahme von Daten aus bildgebenden Verfahren (Röntgenstrahler, MRT oder Computertomographie (CT)) positioniert und unter Druckkontrolle aufgedehnt werden. Die Druckdaten / Pulskurven können vorteilhafterweise auf die Druckmessvorrichtung im Bildsystem der bildgebenden Verfahren projiziert werden. Ferner ist eine ähnliche Ansteuerung der Messpunkte wie bei einer Flussmessung im Herzechodiagramm möglich.

Nach der Untersuchung wird die Druckmessvorrichtung aus dem Gefäß und dem Patient entfernt und die Einstichstelle zum Beispiel durch einen Druckverband verschlossen.

Die Erfindung wird nachfolgend anhand von Ausführungsbeispielen näher erläutert, die in der Zeichnung dargestellt sind. Es zeigen
- FIG. 1: Druckmessvorrichtung mit FBG-Sensoren,
- FIG. 2: Druckmessvorrichtung mit FBG- Sensoren und piezoelektrischen Sensoren,
- FIG. 3: Druckmesssystem in einem biologischen System,
- FIG. 4: Druckmessvorrichtung mit Ummantelung,
- FIG. 5: Anwendungsbeispiel zur epiduralen Druckmessung,
- FIG. 6: Druckmessvorrichtung mit Schutzhülle und atraumatischer Spitze,
- FIG. 7: Schnittdarstellung einer Druckmessvorrichtung mit einem Versteifungsmittel,
- FIG. 8: Anordnung mehrere Messzellenträger in einer Schutzhülle mit Versatz von 1/(Zahl_der_Messzellenträger)-Pitch zueinander, am Beispiel von 3 Messzellenträgern, also 1/3 Pitch-Versatz,
- FIG. 9 bis FIG. 11: Darstellungen eines Fabry-Perot-Interferometers und
- FIG. 12: Schnittdarstellung einer Druckmessvorrichtung mit drei Messzellenträgern.

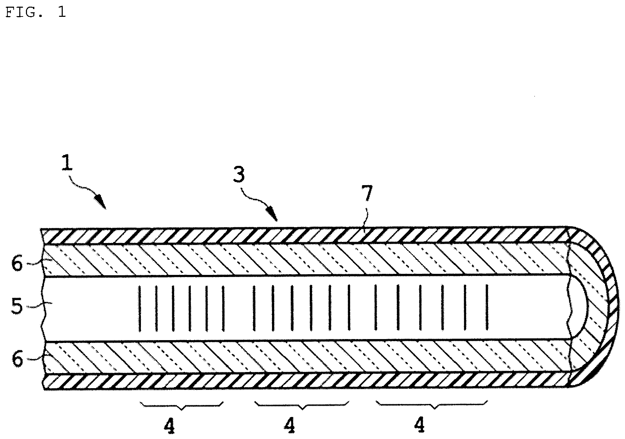

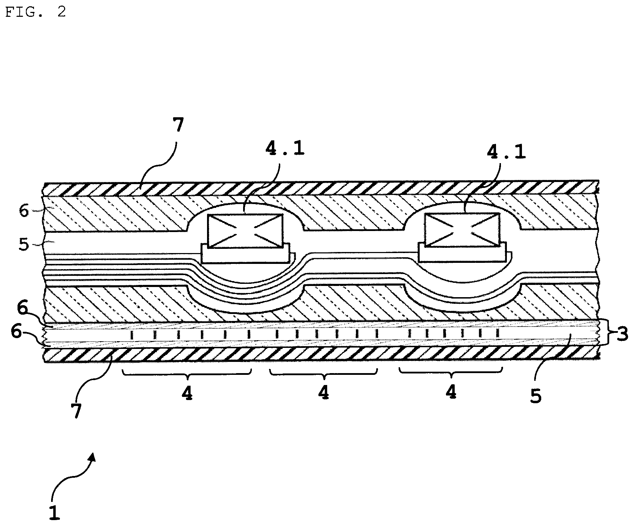

FIG. 1 zeigt eine schematische Darstellung einer Druckmessvorrichtung mit FBG-Sensoren und FIG. 2 eine schematische Darstellung einer Druckmessvorrichtung mit FBG-Sensoren und piezoelektrischen Sensoren. Die Druckmessvorrichtung 1 mit dem Messzellenträger 3 ist biegeflexibel oder biegeelastisch ausgestaltet, so dass eine auf den Messzellenträger 3 radial wirkende Kraft, eine Dehnung der in dem Messzellenträger 3 zueinander beabstandet angeordnete FBG-Sensoren 4 bewirkt. Zusätzlich zu den FBG-Sensoren 4 können auch piezoelektrische Sensoren 4.1 in der Druckmessvorrichtung 1 vorliegen, wobei in FIG. 1 eine Druckmessvorrichtung 1 mit FBG-Sensoren 4 und in FIG. 2 mit piezoelektrischen Sensoren 4.1 dargestellt ist. Die FBG-Sensoren 4 liegen in Form von Gitterstrukturen in einem Messzellenträger 3, der als LWL ausgeführt ist, vor.

Die FBG-Sensoren 4 sind vorzugsweise so gestaltet, dass sie unterschiedliche Gitterstrukturen und folglich unterschiedliche Bragg-Wellenlängen aufweisen und somit unterschiedliche Wellenlängen reflektieren. Hierdurch können die FBG-Sensoren 4 entweder einzeln mit einer entsprechenden Wellenlänge oder mit breitbandigen Lichtquellen angesteuert werden. Sobald eine Kraft auf den Messzellenträger 3 wirkt, kommt es zu einer Dehnung des Messzellenträgers 3, das heißt des FBG-Sensors 4 oder des piezoelektrischen Sensors 4.1. Diese Dehnung bewirkt bei dem FBG-Sensor 4, dass von dem Sensor eine zu der ursprünglichen Bragg-Wellenlänge verschiedene Wellenlänge reflektiert wird, was wiederum erfasst und in einen einwirkenden Druck umgerechnet werden kann.

Im Falle des piezoelektrischen Sensors 4.1, der in einem elektrisch leitenden Kabel angeordnet ist, bilden sich durch die gerichtete Verformung Dipole innerhalb der Elementarzellen (Verschiebung der Ladungs-Schwerpunkte). Die Aufsummierung über das damit verbundene elektrische Feld in allen Elementarzellen des piezoelektrischen Sensors 4.1 führt zu einer messbaren elektrischen Spannung. Es kann eine Vielzahl an piezoelektrischen Sensoren 4.1 in einem Kabel in Reihe geschaltet vorliegen. Durch eine steigende Anzahl an piezoelektrischen Sensoren 4. 1 mit einhergehender Anzahl der Zu- und Ableitungen wächst jedoch auch der Durchmesser der Druckmessvorrichtung 1. Um diesen klein zu halten, können die piezoelektrischen Sensoren 4.1 nacheinander durch ein Bus-System angesprochen werden. Durch die erfindungsgemäßen Messzellen ist eine einfache und zuverlässige Druckmessung in einem biologischen System möglich. Die Druckmessvorrichtung 1 kann einen oder mehrere Arbeitskanäle 9 aufweisen, über die beispielsweise ein Führungsdraht oder ein Versteifungsmittel einführbar oder ein Medikament applizierbar ist.

Um die Empfindlichkeit der Druckmessung zu erhöhen, können die FBG-Sensoren 4 oder in Kombination mit den piezoelektrischen Sensoren 4.1 in verschiedenen Schichten 5, 6 des Messzellenträgers 3 angeordnet sein, so dass beispielsweise eine Dehnung und Stauchung des Messzellenträgers 3 erfassbar ist. Bei der ersten Schicht 5 kann es sich beispielsweise im Falle des LWLs um den Kern und bei der zweiten Schicht 6 um ein weiteres Lichtwellenleitermedium oder den Mantel 7 handeln. Wie beispielhaft FIG. 2 zeigt, umfasst die Druckmessvorrichtung 1 einen Messzellenträger 3 mit mehreren Schichten 5, 6, wobei eine Schicht 5 mit FBG-Sensoren 4 als LWL und die weitere Schicht 5 mit piezoelektrischen Sensoren 4.1 als elektrisches Kabel ausgebildet ist. Die weitere Schicht 6 kann beispielsweise eine Mantelschicht sein. Die Druckmessvorrichtung 1 gemäß der FIG. 2 zeigt zudem eine Schutzschicht 7.

Es ist auch möglich, Messzellenträger zu fertigen, die lediglich einen Kernbereich aufweisen, mit dem dann wiederum Messzellen verbunden sind oder in dem Messzellen angeordnet sind. Solche Messzellenträger weisen in der Regel keinen Mantelbereich auf. Im Falle des Kabels kann die erste Schicht 5 ein Kernbereich sein, in dem die elektrischen Leitungen verlaufen und die zweite Schicht 6 kann ein Mantelbereich des Kabels sein. Weitere Möglichkeiten zur Steigerung der Messempfindlichkeit oder Messsensitivität umfassen beispielsweise ein Abdünnen der LWL durch Ätzen oder durch ein Ausziehen an der Faserspitze, so dass die Fasern als mikrostrukturierte Fasern vorliegen. Hierdurch wird nicht nur die Messsensitivität erhöht, sondern auch die Steifigkeit der LWLs verringert.

Ferner kann der Messzellenträger 3 eine biokompatible Schutzschicht 7 aufweisen. Hierbei kann es sich beispielsweise um eine Kunststoffbeschichtung handeln, die als mechanischer Schutz auf der Oberfläche des Messzellenträgers 3 aufgebracht ist und biokompatibel ausgestaltet ist. Die Druckmessvorrichtung kann zur Druckmessung in flüssigkeitsgefüllten Räumen verwendet werden, in denen zum Beispiel hydrostatische Druck-Schwingungsamplituden gemessen werden. Die FBG-Sensoren 4 liegen in einem Abstand von 500 mm bis 0 mm, vorzugsweise 100 mm bis 3 mm, besonders bevorzugt 8 mm bis 4 mm in dem Messzellenträger 3 vor und weisen eine Länge von circa 1 mm bis circa 500 mm, bevorzugt 2 mm bis 200 mm, besonders bevorzugt 3 mm bis 20 mm auf. Der LWL ist möglichst flexibel ausgestaltet, um den engen und starr vorgegebenen Bedingungen in einem biologischen System folgen zu können.

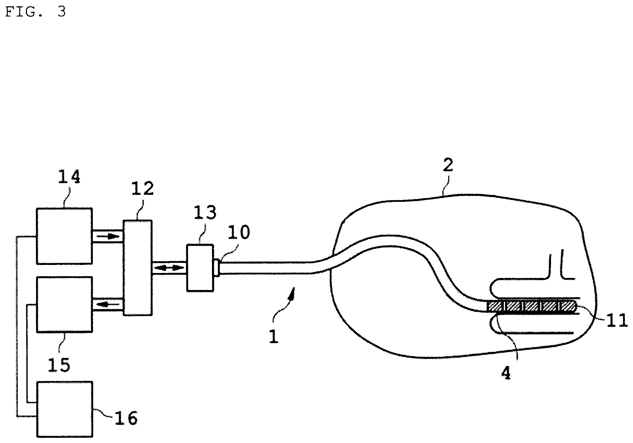

FIG. 3 stellt schematisch ein Druckmesssystem in einem biologischen System dar. Es hat sich überraschenderweise herausgestellt, dass für die erfindungsgemäße Druckmessvorrichtung 1 auch handelsübliche Telekommunikations-LWL mit 125µm oder gar 80µm Faser-Durchmesser zum Einsatz kommen können. Jedoch können auch sonstige Faser-Durchmesser verwendet werden, wie beispielsweise kardiospezifischer Durchmesser von maximal 0,36 mm.

Der Messzellenträger 3 ist am ersten, Auswerteeinheit-nahen, Ende 10 über einen Koppler 12, einen Konnektor 13 oder Zirkulator (nicht dargestellt) mit einer Lichtquelle 14 und einem optischen Sensor 15 verbunden. Die Auswertung, das heißt die Errechnung des Druckes erfolgt mittels einer Datenverarbeitungseinheit 16. Die Ansteuerung der FBG-Sensoren 4 erfolgt über in der Industrie gängige Lichtquellen 14, wie zum Beispiel durchstimmbare schmalbandige Laser oder spektralbreitbandige jeweils fasergekoppelte Lichtquellen. Die Auswertung erfolgt durch industriegängige optische Sensoren 15, je nach eingesetzter Lichtquelle 14 als reiner Photosensor oder als ein System aus Lichtfrequenztrenner und Sensor, zum Beispiel ein Monochromator und eine Charge-Coupled-Device-(CCD)-Zeile. Lichtquelle 14 und Sensor 15 sind durch übliche Systeme, wie optischem Koppler 12 oder Zirkulator (nicht dargestellt), eingekoppelt. Die Druckmessvorrichtung 1 wird durch einen handelsüblichen LWL-Konnektor 13 an das Signalsystem angeschlossen.

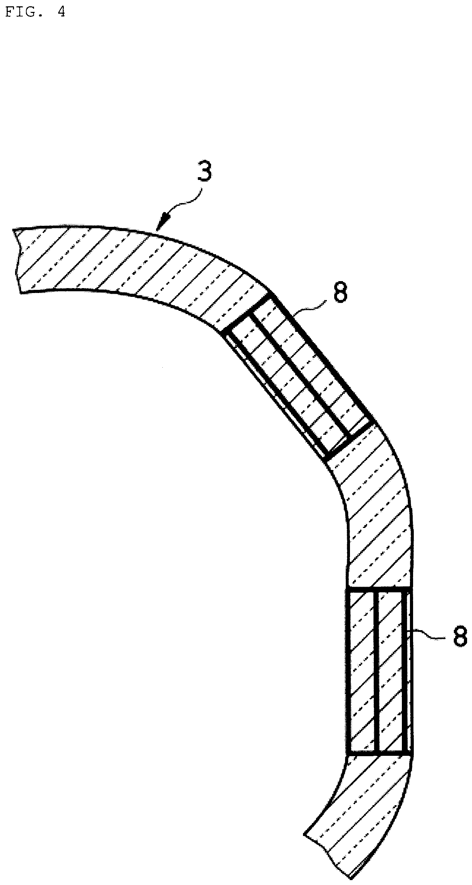

FIG. 4 zeigt eine schematische Darstellung einer Druckmessvorrichtung mit Ummantelung. Es liegt mindestens ein FBG-Sensoren 4 in einem axial nicht flexiblen Bereich 8 des Messzellenträgers 3 als Referenzmesszelle vor. Durch metallische Einbringungen in den Messzellenträger 3 oder durch zusätzliche Ummantelungen, zum Beispiel mit einem Röntgen kontraststreifen, kann die Lage der Druckmessvorrichtung durch zum Beispiel Röntgendurchleuchtung bestimmt werden. Es können auch kugelförmige Metallteilchen beim Extrudieren mit der Extrudierungssubstanz versetzt werden. Die Metallteilchen können auch aus einem Übergangsmetall hergestellt sein. Diese Kügelchen werden zwar beim Röntgen detektiert, erzeugen aber bei einer Kernspinresonanz-Spektroskopie keine nennenswert störende Interferenz. Ferner kann ein Marker in den Messzellenträger 3 integriert werden. Bei dem Marker kann es sich beispielsweise um ein Übergangsmetall, zum Beispiel Tantal handeln, so dass der Einsatz der Druckmessvorrichtung im MRT ermöglicht wird und hier eine Bildgebung erfolgen kann. Die genannten metallischen Einbringungen können auch in Form von Schienen, Hülsen oder Netzen, gegebenenfalls mit Ringen an den Enden, genutzt werden, um der Druckmessvorrichtung stellenweise eine höhere Steifigkeit zu verleihen und damit die Druckmessungen gegen Artefakte durch Biege-, Zug-, Stauch- oder Scherkräfte in der Druckmessvorrichtung zu schützen. Dies kann auch bei Referenzmesszellen zum Einsatz kommen, um die zum Beispiel eine Hülse oder Schienung angeordnet ist, so dass nur die Temperatur in dem biologischen System und nicht die Dehnung des Messzellenträgers eine Auswirkung auf die Eigenschaften der FBG-Sensoren ausübt.

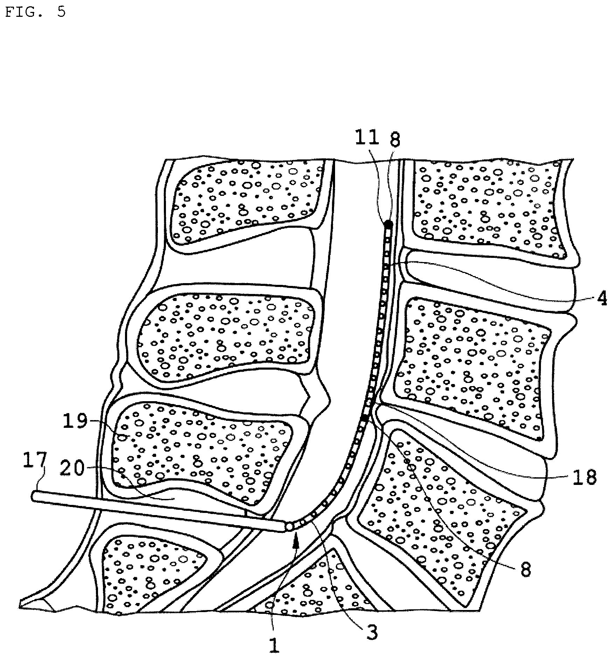

FIG. 5 zeigt ein Anwendungsbeispiel zur epiduralen Druckmessung. Es soll ein Druck zur Diagnose einer zentralen lumbalen Spinalstenose erfasst werden, wobei eine topographische epidurale Druckableitung erfolgt. Die Druckmessvorrichtung 1 wird über eine Epiduralnadel 17 von wenigen Zentimeter bis zu circa einem Meter tief in den Spinalkanal eingeführt, vorzugsweise durch das zwischen den Dornfortsätzen 19 der Lendenwirbelsäule liegende Ligamentum flavum 20. Es können Drücke von 1 bis mindestens 150 mmHg auf einer Messstrecke von 1 bis 30 cm erfasst werden, wobei die FBG-Sensoren 4 in einem Abstand von circa 1 cm in einem circa 0,6 mm dicken Messzellenträger 3 von circa 50 cm Länge und anschließender 5 Meter langer Leitung vorliegen. Die Druckmessvorrichtung 1 kann am zweiten, Auswerteeinheit-fernen, Ende 11 und in einer weiteren Position an dem Messzellenträger 3 Marker, zum Beispiel aus Tantal, 8 aufweisen. Hierdurch kann die Druckmessvorrichtung in einem Röntgen-Bildwandler, im CT oder MRT sichtbar gemacht werden.

Eine aufbereitete Darstellung der abgeleiteten Werte in Bezug auf ein eingelesenes, skaliertes Bild der Lendenwirbelsäule bewirkt, dass sowohl statische und dynamische Einzelmessungen als auch zeitliche Verläufe dokumentiert werden können. Die Druckmessvorrichtung 1 weist Arbeitskanäle (nicht dargestellt) auf, die über entsprechende Ausgänge wie beispielsweise 18 mit kleinen Lumina zur Applikation von Medikamenten vorgesehen sein können. Eine Software erhält kontinuierliche oder diskontinuierliche Messdaten, beispielsweise mit 50 Hz je Messzelle, über den jeweiligen Druckzustand in der Druckmessvorrichtung 1 und erlaubt damit Rückschlüsse auf die Druckverhältnisse relativ zur Lage der Druckmessvorrichtung 1. Über Nativ-Röntgen, ein CT oder MRT kann ergänzend eine absolute Lagebestimmung der Druckmessvorrichtung vorgenommen werden.

Es ist mit der Druckmessvorrichtung eine kontinuierliche Aufzeichnung der atem- / pulssynchronen epiduralen Druckwellen oberhalb, auf Höhe und / oder unterhalb der maximalen Spinalstenose, zum Beispiel als intraoperatives Monitoring der Dekompression des Duralsacks möglich. Anhand der Amplitude der Druckwellen können prä- und intraoperative Indikationsstellung beziehungsweise Indikationsüberprüfung unter Einbeziehung von an die maximale Stenose angrenzenden Nachbarsegmenten, das heißt Ausweitung der Dekompression, festgestellt werden. Ferner können überraschenderweise klinisch bedeutende Engstellen durch eine Differenzbildung in den Amplituden oberhalb des Grunddruckes festgestellt werden.

Mittels der Druckmessvorrichtung sind dynamische und statische Druckmessungen mithilfe von Messungen unter einem Standardstellungs- / Standardbewegungsprogramm, beispielsweise liegen, Sitzen (Vor- / Rückbeugen), Stehen (Vor- / Rückbeugen), Hinlegen/ Aufstehen, Hinsetzen/ Aufstehen, Treppensteigen, Laufbandtest mit definierter Lokalisation der Druckmessvorrichtung und Aufzeichnung mit zum Beispiel einem mobilem Gerät oder Funkübertragung der Daten möglich.

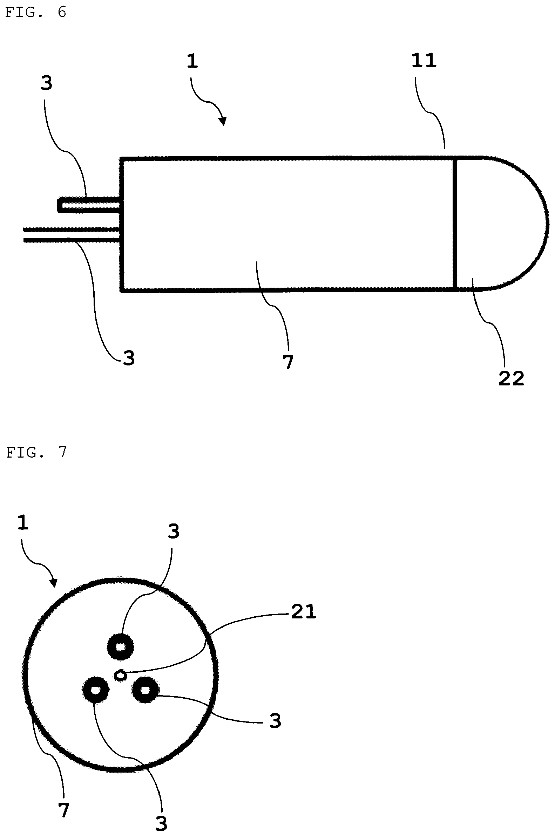

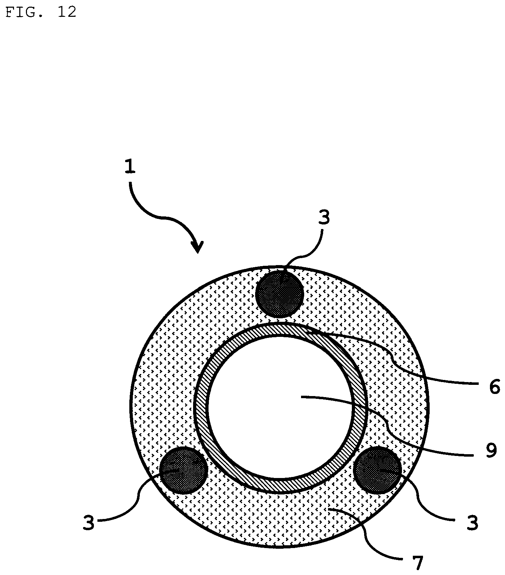

FIG. 6 zeigt eine Druckmessvorrichtung mit Schutzhülle und atraumatischer Spitze und FIG. 7 eine Schnittdarstellung einer Druckmessvorrichtung mit einem Versteifungsmittel. Die Druckmessvorrichtung 1 kann aus mehreren Messzellenträgern 3 bestehen, die von einer Schutzschicht oder Schutzhülle 7 aus beispielsweise Kunststoff umgeben sind. Am Auswerteeinheit-fernen Ende 11 ist eine atraumatische Spitze 22 angeordnet. Die atraumatische Spitze 22 ist aus einem Kunststoffmaterial gefertigt, so dass es zu keiner Verletzung des biologischen Systems beim Einführen und Durchführen der Druckmessvorrichtung 1 kommt. Eine Schnittdarstellung einer Druckmessvorrichtung 1 mit Schutzhülle 7 ist in FIG. 7 und eine Schnittdarstellung einer Druckmessvorrichtung mit drei Messzellenträgern ist in FIG. 12 dargestellt. Mittig in der Schutzhülle 7 ist ein Versteifungsmittel 21 angeordnet. Hierbei kann es sich beispielsweise um ein drahtförmiges Mittel handeln, welches aus aromatischen Polyamiden hergestellt ist. Das Versteifungsmittel 21 kann auch gewebeförmig ausgestaltet sein. Hier bietet sich besonders ein Gewebe aus dem zuvor genannten Kunststoffen an. Es können mehrere Messzellenträger 3 in der Druckmessvorrichtung 1, insbesondere der Schutzhülle 7 vorliegen. Beispielsweise können drei Messzellenträger 3 um einen Arbeitskanäle 9 angeordnet sein, wobei es auch möglich ist, zwei oder mehr als drei Messzellenträger 3 in einer der Schichten der Druckmessvorrichtung anzuordnen. Die zweite Schicht 6 kann als Mantelbereich ausgestaltet sein.

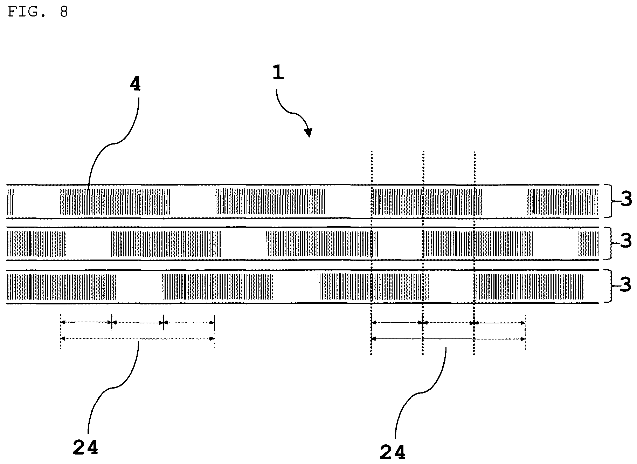

FIG. 8 zeigt eine Anordnung mehrere Messzellenträger in einer Schutzhülle. Die Messzellenträger 3 sind exzentrisch angeordnet und gegeneinander jeweils um 1/n, zum Beispiel im Fall von drei Messzellenträger 1/3 des Pitches 24 der FBG-Sensoren 4 verschoben. Dies erlaubt eine Erkennung, Separierung und Auswertung von biegungs- dehnungs- und radialkraftbedingten Veränderungen der Reflexionswellenlänge der FBG-Sensoren 4 mit besserer Ortsauflösung, als es das Pitch 24 der FBG-Sensoren 4 allein bietet. Auch gibt es keine messfreien Stellen zwischen den FBG-Sensoren 4 durch die Überlappung der Felder. Der Versatz, mit dem die FBG-Sensoren 4 zueinander angeordnet sind, beträgt 1/n des Pitches 24, wobei n die Zahl der Messzellenträger 3 in der Druckmessvorrichtung, insbesondere der Schutzhülle 7 sind. FIG. 8 zeigt eine Ausgestaltung mit drei Messzellenträgern 3, so dass n=3 und der Versatz ein Drittel des Pitches (dem Abstand von Mitte eines FBG-Sensorfeldes zur Mitte des nächsten) beträgt (gekennzeichnet durch die gestrichelten Linien). Es können jedoch auch nur zwei Messzellenträger 3, das heißt LWLs, in einer Schutzhülle 7, beispielsweise einem Mantel um einen Arbeitskanal herum angeordnet sein. Auch mehr als drei Messzellenträger 3 sind möglich. Hierdurch ist eine Laufzeitmessung möglich und es können Störeinflüsse, wie zum Beispiel ein Temperatureinfluss auf die FBG-Sensoren, kompensiert werden. Zudem kann in den, zwischen den Messzellenträger 3 angeordneten Arbeitskanal, ein Versteifungs- oder Führungsmittel eingeführt werden, mit dem die Steifigkeit der Druckmessvorrichtung an die jeweilige Verwendung der Druckmessvorrichtung angepasst wird, indem Versteifungs- oder Führungsmittel mit unterschiedlichen Steifigkeiten herangezogen werden.

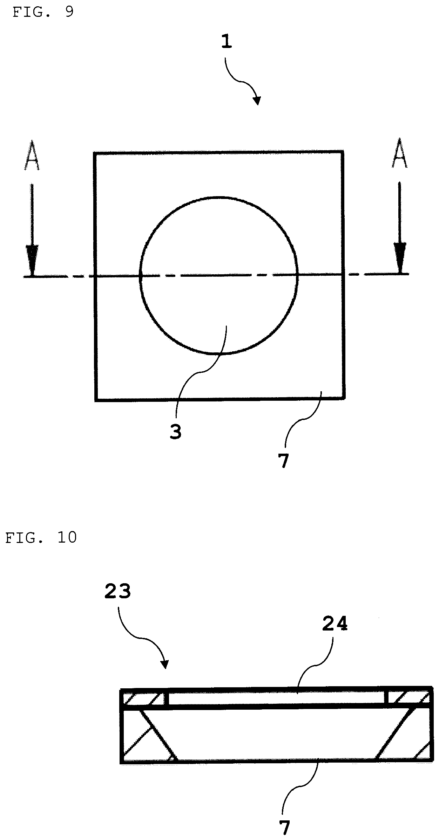

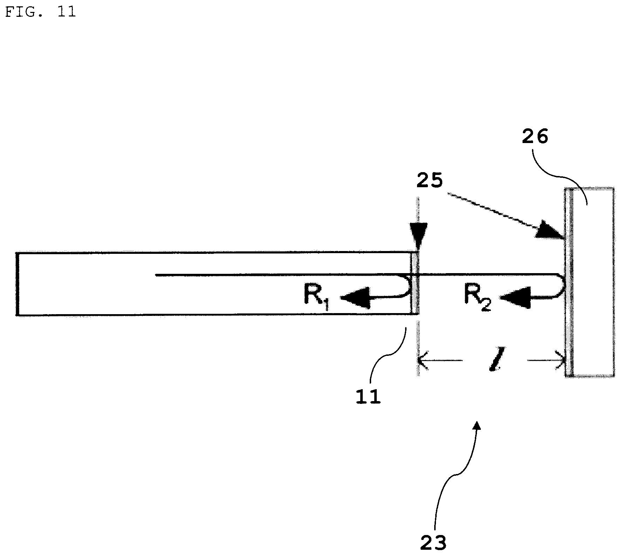

FIG. 9, FIG. 10 und FIG. 11 zeigen Darstellungen eines Fabry-Perot-Interferometers in einer, beispielsweise atraumatischen, Spitze. In FIG. 9 ist der Querschnitt einer Druckmessvorrichtung 1 mit einem Messzellenträger 3 und einer Schutzhülle dargestellt. Hierbei soll es sich um das Auswerteeinheit-ferne Ende mit einer atraumatischen Spitze handeln. Die Schnittdarstellung (A-A) mit dem in der Spitze 22 vorliegenden Fabry-Perot-Interferometer 23 ist in FIG. 10 zu erkennen und eine schematische Seitenansicht desselben in FIG. 11. In der Spitze 22 befindet sich ein, beispielsweise in MEMS-Technologie gefertigtes, Fabry-Perot-Interferometer 23, welches sich aus mindestens einem Ende eines Messzellenträgers, insbesondere eines LWLs 3 (Auswerteeinheit-fernes Ende 11) sowie einer, durch Druckeinwirkung und daraus folgender Verformung der Spitze 22, deformierbaren verspiegelten Membran 24 zusammensetzt.

Dieses Fabry-Perot-Interferometer 23 bildet einen Drucksensor, welcher beispielsweise im gleichen Wellenlängenfenster auswertbar ist, wie die FBG-Sensoren. FIG. 11 zeigt das allgemeine Funktionsprinzip eines Fabry-Perot-Interferometers 23. Am LWL Ende 11 sind zwei teilreflektierende Spiegel 25 angeordnet, die Lichtstrahlen (R1 und R2) reflektieren. Zwischen den Spiegeln 25 ist ein Abstand I. Das Transmissionsspektrum dieser Anordnung zeigt schmale Transmissions-Maxima für Wellenlängen, welche die Resonanzbedingung erfüllen, während andere Spektralbereiche in der Transmission nahezu vollständig ausgelöscht werden. Dies geschieht durch konstruktive oder destruktive Interferenz der Teilstrahlen. Dadurch, dass die verspiegelte Membran 24 deformierbar ausgestaltet ist, bewirkt eine einwirkende Kraft (Druck) eine Änderung des Transmissionsspektrums und folglich der reflektierten Strahlen, so dass auch geringe Druckänderungen an der Spitze 22 messbar sind. Dies ist insbesondere beim Einführen der Druckmessvorrichtung 1 in ein biologisches System und bei dem Durchführen der Druckmessvorrichtung 1 durch dieses vorteilhaft sowie zum Kalibrieren der Druckmessvorrichtung auf Temperaturartefakte sowie zum Messen von anderen Druckbereichen und anderen Druckauflösungen, ggf. auch in anderen Messungsfrequenzen.

## Patentansprüche

1. Druckmessvorrichtung (1) zur Druckmessung in einem biologischen System (2) mit einem biegeflexibel oder biegeelastisch ausgestalteten und aus mindestens einem Lichtwellenleiter bestehenden Messzellenträger (3), der ein Auswerteeinheit-nahes Ende (10) und ein Auswerteeinheit-fernes Ende (11) aufweist, **dadurch gekennzeichnet, dass** in dem Messzellenträger (3) mindestens zwei zueinander beabstandet angeordnete Faser-Bragg-Gitter Sensoren (4) vorliegen und in dem Auswerteeinheit-fernen Ende (11) ein Fabry-Perot-Interferometer (23) angeordnet ist, welches sich aus mindestens einem Ende des Auswerteeinheit-fernen Endes (11) des Messzellenträgers (3) sowie mindestens einer deformierbaren verspiegelten Membran (24) zusammensetzt, wobei das Fabry-Perot-Interferometer (23) im gleichen Wellenlängenfenster wie die Faser-Bragg-Gitter Sensoren (4) auswertbar ist.

2. Druckmessvorrichtung (1) nach Anspruch 1, **dadurch gekennzeichnet, dass** der Messzellenträger (3) mindestens zwei Schichten (5, 6) aufweist und die Faser-Bragg-Gitter Sensoren (4) in einer ersten (5) und / oder einer zweiten Schicht (6) angeordnet sind.

3. Druckmessvorrichtung (1) nach Anspruch 1 oder 2, **dadurch gekennzeichnet, dass** der Messzeilenträger (3) eine äußere aus einem Kunststoff bestehende Schutzschicht (7) aufweist.

4. Druckmessvorrichtung (1) nach einem oder mehreren der vorhergehenden Ansprüche, **dadurch gekennzeichnet, dass** der Lichtwellenleiter eine polymere optische Faser oder eine Glasfaser ist.

5. Druckmessvorrichtung (1) nach einem oder mehreren der vorhergehenden Ansprüche, **dadurch gekennzeichnet, dass** der Messzellenträger (3) einen piezoelektrischer Sensor (4.1) in der ersten und / oder zweiten Schicht (5, 6) aufweist.

6. Druckmessvorrichtung (1) nach einem oder mehreren der vorhergehenden Ansprüche, **dadurch gekennzeichnet, dass** mehrere in dem Messzellenträger (3) vorliegende Faser-Bragg-Gitter-Sensoren (4) unterschiedliche Gitterstrukturen und folglich unterschiedliche Bragg-Wellenlängen aufweisen, so dass sie unterschiedliche Wellenlängen reflektieren.

7. Druckmessvorrichtung (1) nach einem oder mehreren der vorhergehenden Ansprüche, **dadurch gekennzeichnet, dass** mindestens ein Faser-Bragg-Gitter Sensor (4) in einem axial nicht flexiblen Bereich (8) des Messzellenträgers (3) als Referenzmesszelle vorliegt.

8. Druckmessvorrichtung (1) nach einem oder mehreren der vorhergehenden Ansprüche, **dadurch gekennzeichnet, dass** der Messzellenträger (3) mindestens einen Marker aus einem Metall oder Übergangsmetall (8) aufweist, der in oder an einer Schicht (5, 6) des Messzellenträgers (3) vorliegt.

9. Druckmessvorrichtung (1) nach einem oder mehreren der vorhergehenden Ansprüche, **dadurch gekennzeichnet, dass** der Messzellenträger (3) mindestens einen Arbeitskanal (9) mit einem Eingang und Ausgang aufweist.

10. Druckmessvorrichtung (1) nach einem oder mehreren der vorhergehenden Ansprüche, **dadurch gekennzeichnet, dass** am Auswerteeinheit-fernen Ende (11) eine atraumatische Spitze (22) aus einem Kunststoff, insbesondere Polyoxymethylen, Polyurethan und/oder Polyamid oder eine Führungsstruktur vorliegt.

11. Druckmessvorrichtung (1) nach einem oder mehreren der vorhergehenden Ansprüche, **dadurch gekennzeichnet, dass** der Messzellenträger (3) am ersten, Auswerteeinheit-nahen, Ende (10) über einen Koppler (12), einen Konnektor (13) oder Zirkulator mit einer Lichtquelle (14) und einem optischen Sensor (15) verbunden ist.

12. Druckmessvorrichtung (1) nach einem oder mehreren der vorhergehenden Ansprüche, **dadurch gekennzeichnet, dass** mindestens zwei Messzellenträger (3) in der Schutzschicht (7) angeordnet sind.

13. Druckmessvorrichtung (1) nach einem oder mehreren der vorhergehenden Ansprüche, **dadurch gekennzeichnet, dass** die Messzellenträger (3) in Längsrichtung der Schutzschicht (7) versetzt zueinander angeordnet sind.

14. Druckmessvorrichtung (1) nach einem oder mehreren der vorhergehenden Ansprüche, **dadurch gekennzeichnet, dass** die Messzellenträger (3) zueinander um 1/n eines Pitches eines FBG-Sensors (4.1) versetzt zueinander angeordnet sind und n die Anzahl der Messzellenträger (3) in der Schutzschicht (7) ist.

15. Druckmessvorrichtung (1) nach einem oder mehreren der vorhergehenden Ansprüche, **dadurch gekennzeichnet, dass** die Druckmessvorrichtung (1) im Arbeitskanal (9) oder der Schutzschicht (7) ein drahtförmiges oder gewebeförmiges Versteifungsmittel (21) zur Versteifung der Druckmessvorrichtung (1) aufweist.

## Claims

1. Pressure measuring device (1) for measuring pressure in a biological system (2), comprising a flexibly or resiliently designed measuring cell holder (3) and consisting of at least one optical waveguide, which comprises a first end (10) which is close to the evaluation unit, and a second end (11) which is remote from the evaluation unit, **characterised in that**, in the measuring cell holder (3), there are at least two fibre Bragg grating sensors (4) which are arranged at a distance from one another, and wherein a Fabry-Perot interferometer (23) is arranged in the end (11) which is remote from the evaluation unit, which interferometer is composed of at least one end of the second end (11) which is remote from the evaluation unit of the measuring cell holder (3) and at least one mirrored membrane 24 which can be deformed, wherein the Fabry-Perot interferometer (23) can be evaluated in the same wavelength window as the fibre Bragg grating sensors (4).

2. Pressure measuring device (1) according to claim 1, **characterised in that** the measuring cell holder (3) comprises at least two layers (5, 6), and the fibre Bragg grating sensors (4) are arranged in a first (5) and/or second (6) layer.

3. Pressure measuring device (1) according to either claim 1 or claim 2, **characterised in that** the measuring cell holder (3) comprises an outer protective layer (7) consisting of a plastic material.

4. Pressure measuring device (1) according to one or more of the preceding claims, **characterised in that** the optical waveguide is a polymer optical fibre or a glass fibre.

5. Pressure measuring device (1) according to one or more of the preceding claims, **characterised in that** the measuring cell holder (3) comprises a piezoelectric sensor (4.1) in the first and/or second layer (5, 6).

6. Pressure measuring device (1) according to one or more of the preceding claims, **characterised in that** a plurality of fibre Bragg grating sensors (4) which are present in the measuring cell holder (3) have different grating structures and consequently different Bragg wavelengths and thus reflect different wavelengths.

7. Pressure measuring device (1) according to one or more of the preceding claims, **characterised in that** at least one fibre Bragg grating sensor (4) is present in an axially inflexible region (8) of the measuring cell holder (3) as a reference measuring cell.

8. Pressure measuring device (1) according to one or more of the preceding claims, **characterised in that** the measuring cell holder (3) comprises at least one marker made of a metal or transition metal (8) which is present in or on a layer (5, 6) of the measuring cell holder (3).

9. Pressure measuring device (1) according to one or more of the preceding claims, **characterised in that** the measuring cell holder (3) comprises at least one working channel (9) having an input and an output.

10. Pressure measuring device (1) according to one or more of the preceding claims, **characterised in that** an atraumatic tip (22) consisting of a plastic material, especially polyoxymethylene, polyurethane and/or polyamide or a guiding structure is present at the second end (11) which is remote from the evaluation unit.

11. Pressure measuring device (1) according to one or more of the preceding claims, **characterised in that** the measuring cell holder (3) is connected at the first end (10), which is close to the evaluation unit, to a light source (14) and an optical sensor (15) by a coupler (12), a connector (13) or circulator.

12. Pressure measuring device (1) according to one or more of the preceding claims, **characterised in that** at least two measuring cell holders (3) are arranged in the protective layer (7).

13. Pressure measuring device (1) according to one or more of the preceding claims, **characterised in that** the measuring cell holders (3) are arranged in the longitudinal direction of the protective layer (7) so as to be offset from one another.

14. Pressure measuring device (1) according to one or more of the preceding claims, **characterised in that** the measuring cell holders (3) are arranged so as to be offset from one another by 1/n of a pitch of an FBG sensor (4.1), and n is the number of the measuring cell holders (3) in the protective layer (7).

15. Pressure measuring device (1) according to one or more of the preceding claims, **characterised in that** the pressure measuring device (1) comprises a wire-shaped or web-shaped stiffening means (21) in the working channel (9) or the protective layer (7) for stiffening the pressure measuring device (1).

## Revendications

1. Dispositif de mesure de pression (1) pour mesurer la pression dans un système biologique (2), comprenant un support de cellule de mesure (3) qui est flexible ou élastique et composé d'au moins un guide d'ondes optique et qui présente une extrémité (10) proche de l'unité d'évaluation et une extrémité (11) éloignée de l'unité d'évaluation, **caractérisé en ce qu'**au moins deux capteurs à réseau de Bragg sur fibre (4) disposés à distance l'un de l'autre sont présents dans le support de cellule de mesure (3), et un interféromètre Fabry-Perot (23) est disposé dans l'extrémité (11) éloignée de l'unité d'évaluation, lequel interféromètre est composé d'au moins une extrémité de l'extrémité (11) du support de cellule de mesure (3) éloignée de l'unité d'évaluation et d'au moins une membrane réfléchissante déformable (24), l'interféromètre de Fabry-Perot (23) pouvant être exploité dans la même fenêtre de longueurs d'onde que les capteurs à réseau de Bragg sur fibre (4).

2. Dispositif de mesure de pression (1) selon la revendication 1, **caractérisé en ce que** le support de cellule de mesure (3) est muni d'au moins deux couches (5, 6), et les capteurs à réseau de Bragg sur fibre (4) sont disposés dans une première (5) et/ou une deuxième couche (6).

3. Dispositif de mesure de pression (1) selon la revendication 1 ou 2, **caractérisé en ce que** le support de cellule de mesure (3) est muni d'une couche de protection extérieure (7) en matière plastique.

4. Dispositif de mesure de pression (1) selon l'une ou plusieurs des revendications précédentes, **caractérisé en ce que** le guide d'onde optique est une fibre optique polymère ou une fibre de verre.

5. Dispositif de mesure de pression (1) selon l'une ou plusieurs des revendications précédentes, **caractérisé en ce que** le support de cellule de mesure (3) est muni d'un capteur piézoélectrique (4.1) dans la première et/ou la deuxième couche (5, 6).

6. Dispositif de mesure de pression (1) selon l'une ou plusieurs des revendications précédentes, **caractérisé en ce que** plusieurs capteurs à réseau de Bragg sur fibre (4) présents dans le support de cellule de mesure (3) présentent des structures de réseau différentes et par conséquent des longueurs d'onde de Bragg différentes de sorte qu'ils réfléchissent longueurs d'onde différentes.

7. Dispositif de mesure de pression (1) selon l'une ou plusieurs des revendications précédentes, **caractérisé en ce qu'**au moins un capteur à réseau de Bragg sur fibre (4) est présent en tant que cellule de mesure de référence dans une zone non flexible axialement (8) du support de cellule de mesure (3).

8. Dispositif de mesure de pression (1) selon l'une ou plusieurs des revendications précédentes, **caractérisé en ce que** le support de cellule de mesure (3) est muni d'au moins un marqueur en métal ou en métal de transition (8) qui est présent dans ou sur une couche (5, 6) du support de cellule de mesure (3).

9. Dispositif de mesure de pression (1) selon l'une ou plusieurs des revendications précédentes, **caractérisé en ce que** le support de cellule de mesure (3) est muni d'au moins un canal de travail (9) avec une entrée et une sortie.

10. Dispositif de mesure de pression (1) selon l'une ou plusieurs des revendications précédentes, **caractérisé en ce que** l'extrémité (11) éloignée de l'unité d'évaluation présente une pointe atraumatique (22) en matière plastique, en particulier en polyoxyméthylène, en polyuréthane et/ou en polyamide, ou une structure de guidage.

11. Dispositif de mesure de pression (1) selon l'une ou plusieurs des revendications précédentes, **caractérisé en ce qu'**au niveau de la première extrémité (10) proche de l'unité d'évaluation, le support de cellule de mesure (3) est relié à une source lumineuse (14) et à un capteur optique (15) via un coupleur (12), un connecteur (13) ou un circulateur.

12. Dispositif de mesure de pression (1) selon l'une ou plusieurs des revendications précédentes, **caractérisé en ce qu'**au moins deux supports de cellule de mesure (3) sont disposés dans la couche de protection (7).

13. Dispositif de mesure de pression (1) selon l'une ou plusieurs des revendications précédentes, **caractérisé en ce que** les supports de cellule de mesure (3) sont disposés décalés les uns par rapport aux autres dans la direction longitudinale de la couche de protection (7).

14. Dispositif de mesure de pression (1) selon l'une ou plusieurs des revendications précédentes, **caractérisé en ce que** les supports de cellule de mesure (3) sont disposés décalés les uns par rapport aux autres de 1/n d'un pas d'un capteur FBG (4.1), et n est le nombre de supports de cellule de mesure (3) dans la couche de protection (7).

15. Dispositif de mesure de pression (1) selon l'une ou plusieurs des revendications précédentes, **caractérisé en ce que** le dispositif de mesure de pression (1), dans le canal de travail (9) ou dans la couche de protection (7), est muni d'un moyen de raidissement (21) en forme de fil ou de tissu pour raidir le dispositif de mesure de pression (1).
